# EUROPEAN PATENT APPLICATION

(11) **EP 3 866 172 A1**
(43) Date of publication of application: **18.08.2021**
(21) Application number: 19870784.6
(22) Date of filing: 11.10.2019
(51) Int. Cl.: G16H 20/00

(54) **METHOD, DEVICE, AND PROGRAM FOR ASSESSING RELEVANCE OF RESPECTIVE PREVENTIVE INTERVENTIONAL ACTIONS TO HEALTH IN HEALTH DOMAIN OF INTEREST**

(30) Priority: 12.10.2018 JP 2018193374
(71) Applicant: SUMITOMO DAINIPPON PHARMA CO., LTD., Osaka-shi, Osaka 541 0045 (JP)
(72) Inventor: OCHIAI Yasushi, Osaka-shi, Osaka 554-0022 (JP); YOSHIMIZU Minoru, Osaka-shi, Osaka 554-0022 (JP); MATONO Mitsuhiro, Suita-shi, Osaka 564-0053 (JP)
(74) Representative: Reeve, Nicholas Edward
(86) International application number: PCT/JP2019/040230
(87) International publication number: WO 2020/075842

(57) **Abstract**

Provided is a method for assessing a correlation degree and an influence degree between a health degree in a health domain of concern and each preventive interventional action, based on biological information acquired in a time-series manner. The method comprises: acquiring biological information of an individual in a time-series manner; performing, based on the acquired biological information, an assessment of a health degree of the individual in a time-series manner; acquiring an intervention amount of each of one or more preventive interventional actions in a time-series manner; deriving a correlation degree and an influence degree between the time-series intervention amount of each of the one or more preventive interventional actions and the time-series health degree of the individual; and determining, as a relevant preventive interventional action, a preventive interventional action whose correlation degree is a given value or more, among the one or more preventive interventional actions.

## Description

### TECHNICAL FIELD

The present invention relates to a method for assessing a relevance between a health degree in a health domain of concern and each preventive interventional action, and more specifically to a method for assessing a correlation degree and an influence degree between a health degree in a health domain of concern and each preventive interventional action, based on biological information acquired in a time-series manner.

### BACKGROUND ART

It has increasingly become common to conduct an assessment about a health degree of an individual (person), based on biological information acquired from the living body. The use of this technique allows an individual to know an assessment of his/her own health degree, and a change in the health degree, at home. As one example of a conventional technique, there is a technique relating to a proposal device which comprises: a receiving part to receive user information about a user, acquired by a sensor; an estimation part to estimate a health state of the user, based on the user information received by the receiving part; and a proposal part to propose an insurance to the user, based on the health state estimated by the estimation part (the below-mentioned Patent Document 1). In the technique, a terminal device is configured to measure a wake-up time, a bedtime, a body weight, a body fat percentage, a smoking status, a blood pressure, a heart rate, etc., and transmit the measured data as the user information to the proposal device, and the proposal device is configured to: receive and store the user information; calculate, based on the user information, a health risk score which is an index indicative of a risk of the user in terms of health; and propose an insurance to the user, based on a health state estimated from the calculated health risk score. This technique makes it possible to propose an insurance, based on information about a health risk, obtained from information measured by the user himself/herself. However, this technique is not configured to propose, to the user, what kind of action the user should take to reduce a health risk.

On the other hand, it is often the case that an individual has a health domain of concern, i.e., a health domain about which the individual is particularly concern. Further, an individual is normally interested in an action having a positive effect on health, i.e., an action effective in maintaining/improving the degree of his/her own health (health degree). Hereinafter, an action intended to maintain/improve the health degree in a certain health domain of concern will be referred to as "preventive interventional action". As the preventive interventional action, various types are conceivable. Here, it is generally considered that which of the various types of preventive interventional actions is effective in maintaining/improving the health degree in a health domain of concern of a certain individual varies among individuals, due to a difference in physical makeup, lifestyle habit or the like among individuals. Further, on the assumption that the amount of a preventive interventional action is referred to as "intervention amount", allowing an individual to know an adequate intervention amount is considered to be important in order to allow the individual to effectively perform the preventive interventional action. Here, it is generally considered that how a certain intervention amount of a preventive interventional action is effective in improving the health degree in a health domain of concern also varies among individuals.

### CITATION LIST

### [Parent Document]

Patent Document 1: JP-A 2017-027157

### SUMMARY OF INVENTION

### [Technical Problem]

As mentioned above, the type of preventive interventional action effective in increasing the health degree in a health domain of concern of an individual is important information for maintaining/improving the health degree of the individual. However, it has been impossible to readily derive this information. In order to derive such information, it is considered that there is the need to monitor the health degree in the health domain of concern of the individual in a time-series manner. However, it has been difficult for an individual to derive the health degree readily and objectively. For realizing the monitoring, it is necessary to allow measurement of the health degree using biological information to be performed at home without going to a facility such as a medical center.

Further, an adequate intervention amount of a preventive interventional action for the individual can also be important information for maintaining/improving the health degree of the individual. However, there has not been any technique of, based on recognizing a need to derive the adequate intervention amount, as a problem to be solved, determining an adequate intervention amount for each individual. Here, for deriving an adequate intervention amount of a preventive interventional action for an individual, time-series information about the intervention amount of the preventive interventional action performed by the individual is also required. This is because it is impossible to derive how the preventive interventional action contributes to improvement in the health degree, without such information. For this purpose, it is necessary to monitor the amount of activity of a preventive interventional action usually performed by the individual. Thus, a technique capable of allowing an individual to conveniently monitor biological information at home and check a change in the health degree based on the biological information, and to monitor respective intervention amounts of various preventive interventional actions performed by the individual and check how positive influence each of the preventive interventional actions exerts on the health degree is considered to be very useful in knowing an adequate preventive interventional action for maintaining/improvement of the healthy degree in a health domain of concern of the individual and an optimal amount of the adequate preventive interventional action. However, there has not been such a technique, and thereby it has been impossible for an individual to known an adequate preventive interventional action and the amount thereof by himself/herself.

The present invention has been made in view of the above problem, and is directed to providing a method for assessing a correlation degree and an influence degree between a health degree in a health domain of concern and each preventive interventional action, based on biological information acquired in a time-series manner.

### [Solution to Technical Problem]

The present invention provides a method for assessing, based on one or more pieces of biological information of an individual each deemed to be relevant to a health degree in a health domain of concern of the individual, a relevance between the health degree and each of one or more preventive interventional actions which are likely to contribute to maintaining or improving the health degree of the individual. The method is characterized in that it comprises: acquiring the biological information of the individual in a time-series manner; performing, based on the acquired biological information, an assessment of the health degree of the individual in a time-series manner; acquiring an intervention amount of each of the one or more preventive interventional actions in a time-series manner; deriving a correlation degree and an influence degree between the time-series intervention amount of each of the one or more preventive interventional actions and the time-series health degree of the individual; and determining, as a relevant preventive interventional action, a preventive interventional action whose correlation degree is a given value or more, among the one or more preventive interventional actions.

The method of the present invention may further comprise determining an optimal intervention amount of the relevant preventive interventional action, based on respective values of the influence degree and the intervention amount in the relevant preventive interventional action. In the above method, the optimal intervention amount may be determined as a value of the intervention amount at a time when an amount of increase in the health degree with respect to an increase in the intervention amount becomes equal to or less than a given lower limit. In the method of the present invention, the health degree may have a given threshold above or below which a risk of development of a given disease increases, wherein the method may further comprise predicting a time period before the health degree reaches the threshold, based on a time-series change in the health degree. In the method of the present invention, the correlation degree and the influence degree may be derived by a statistical method. In the method of the present invention, the intervention amount may be an activity amount in terms of a given activity of the individual. In the method of the present invention, the biological information of the individual may include a brain wave, and the health degree of the individual may relate to a cognition function. In the above method, the brain wave may be acquired in a state in which a specific stimulus is applied to the individual. In the above method, the brain wave may be composed of two types of brain waves acquired in a state in which two types of specific stimuli are applied to the individual respectively, wherein the health degree of the individual related to the cognition function may be determined based on a difference in a rate of an intensity of a specific frequency component to a total intensity between the two types of acquired brain waves. In the above method, the two types of stimuli may be music and photographic image, wherein the specific frequency component of the brain waves may correspond to an α2 wave. In the above method, the stimulus may be selected from the group consisting of stimulation-free condition during wakefulness and rest, stimulation-free condition during sleep, sound, music, printed image, photographic image, video image, questionnaire, quiz, game and brain training. In the method of the present invention, the biological information of the individual may be acquired by a wearable terminal.

In the method of the present invention, the intervention amount may include at least one selected from the group consisting of: an amount of walking; an exercise amount of a given exercise; an amount of implementation of a given cognitive training; a sleep time period; an amount of alcohol intake; a number of cigarettes smoked; an amount of nutrition intake; an amount of medication/supplement/health food intake; an amount of auditory stimulation by a given sound or music piece; an amount of visual stimulation by a given still or moving image; and an amount of electrical or magnetic stimulation to the brain. The present invention can also be configured as a device for implementing the above method, a computer program for allowing a computer to function as the device, and a computer readable recording medium storing therein such a computer program.

The present invention further provides a method for assessing, based on one or more pieces of biological information of each of a plurality of individuals each deemed to be relevant to a health degree in a health domain of concern of each of the plurality of individuals, a relevance between the health degree and each of one or more preventive interventional actions which are likely to contribute to maintaining or improving the health degree of each of the plurality of individuals. The method is characterized in that it comprises: acquiring the biological information of each of the plurality of individuals in a time-series manner; performing, based on the acquired biological information, an assessment of the health degree of each of the plurality of individuals in a time-series manner; acquiring an intervention amount of each of the one or more preventive interventional actions for each of the plurality of individuals, in a time-series manner; and deriving a relevance between each of the one or more preventive interventional actions and the health degree, by machine learning using the time-series intervention amount of each of the one or more preventive interventional actions for each of the plurality of individuals and the time-series health degree of a corresponding one of the plurality of individuals.

### [Effect of Invention]

The method of the present invention comprises: acquiring one or more pieces of biological information of an individual each deemed to be relevant to a health degree in a health domain of concern of the individual, in a time-series manner; performing, based on the acquired biological information, an assessment of the health degree of the individual in a time-series manner; acquiring an intervention amount of each of one or more preventive interventional actions which are likely to contribute to maintaining or improving the health degree of the individual, in a time-series manner; deriving a correlation degree and an influence degree between the time-series intervention amount of each of the one or more preventive interventional actions and the time-series health degree of the individual; and determining, as a relevant preventive interventional action, a preventive interventional action whose correlation degree is a given value or more, among the one or more preventive interventional actions. According to this feature, the present invention has an advantageous effect of being able to assess, based on the biological information, a relevance between the health degree and each of the one or more preventive interventional actions.

### BRIEF DESCRIPTION OF DRAWINGS

FIG. 1 is a block diagram showing the configuration of a terminal device 100.
FIG. 2 is an operation flow of the terminal device 100.
FIG. 3 is a conceptual diagram of a scene where a brain wave is acquired in a state in which music is provided to a user.
FIG. 4 is a conceptual diagram of a scene where a brain wave is acquired in a state in which a photographic image is provided to the user.
FIG. 5 is a table showing a list of three items: a health domain of concern; biological information; and sensor device.
FIG. 6 is a chart representing an example of a power spectrum corresponding to a brain wave.
FIG. 7 is a graph cited from the graph of Alpha 2 in Fig. 4 of a research paper authored by Hiroshi YOSHIMURA, et al., wherein the graph shows the relationship between the HDS-R and the amount of change in occupancy rate of an α2 wave.
FIG. 8 is a graph cited from FIG. 2 of a research paper authored by Shinji KATO, et al., wherein the graph shows the relationship between an HDS-R score and an MMSE score.
FIG. 9 is a table representing the relationship between the amount of change in occupancy rate of the α2 wave and the amount of change in the MMSE score.
FIG. 10 is a graph representing an example of a brain wave-related index having a significant relationship with the MMSE score.
FIG. 11 is a table representing examples of a preventive interventional action and intervention amounts thereof acquired in a time-series manner.
FIG. 12 illustrates a table showing an example of a result of multi-regression analysis.
FIG. 13 illustrates graphs cited from FIGURE 3 of a research paper authored by Charis Styliadis, et al., wherein FIG. 13(a) shows the relationship between a difference in the MMSE score and a difference in δ wave intensity, and FIG. 13(b) shows the relationship between a difference in the MMSE score and a difference in θ wave intensity.
FIG. 14 is a graph representing an example of the relationship between the intervention amount and a health degree.

### DESCRIPTION OF EMBODIMENTS

### (Concept of Invention)

The present invention is configured to derive a health degree in a health domain of concern from biological information acquired using a sensor device capable of allowing a user to perform measurements at home, while monitoring an intervention amount of a preventive interventional action, thereby deriving a correlation degree between the intervention amount of the preventive interventional action and the health degree, and an influence degree of the preventive interventional action. Examples of the health domain of concern, biological information relevant to the health domain of concern, and the sensor device for acquiring the biological information are as follows.

Firstly, the health domain of concern may be dementia. In this case, as the biological information, it is possible to use information derived from brain activity (brain wave, cerebral blood flow, magnetoencephalography, etc.), information derived from voice (conversational content, articulation, intonation, etc.), etc. Further, as the sensor device for acquiring the biological information, it is possible to use an electroencephalograph, an NIRS brain measurement device, a magnetoencephalograph, a voice analyzer, etc.

Secondly, the health domain of concern may be depression (ademonia). In this case, as the biological information, it is possible to use information derived from brain activity (brain wave, cerebral blood flow, magnetoencephalography, etc.), information derived from autonomic nerves, information derived from voice (conversational content, articulation, intonation, etc.), etc. Further, as the sensor device for acquiring the biological information, it is possible to use an electroencephalograph, an NIRS brain measurement device, a magnetoencephalograph, a heart rate meter, a voice analyzer, etc.

Thirdly, the health domain of concern may be stroke (apoplexy). In this case, as the biological information, it is possible to use vascular age, etc. Further, as the sensor device for acquiring the biological information, it is possible to use a blood pressure meter (sphygmomanometer), an acceleration pulse wave meter (acceleration plethysmograph), a pulse wave-blood pressure meter, etc.

Fourthly, the health domain of concern may be arthrosis. In this case, as the biological information, it is possible to use information derived from walking pattern (because deformation of a joint appears in walking pattern), etc. Further, as the sensor device for acquiring the biological information, it is possible to use an acceleration sensor, etc.

Fifthly, the health domain of concern may be diabetes. In this case, as the biological information, it is possible to use information derived from blood glucose level, autonomic nerves, etc. Further, as the sensor device for acquiring the biological information, it is possible to use a blood glucose meter, a heart rate meter, a heart rate variability sensor, etc.

Sixthly, the health domain of concern may be bone fracture or fall. In this case, as the biological information, it is possible to use bone density, muscle mass, etc. Further, as the sensor device for acquiring the biological information, it is possible to use a body composition analyzer, etc.

Seventhly, the health domain of concern may be hypertension (hyperpiesia). In this case, as the biological information, it is possible to use blood pressure, etc. Further, as the sensor device for acquiring the biological information, it is possible to use a blood pressure meter, etc.

Eighthly, the health domain of concern may be lipid abnormality. In this case, as the biological information, it is possible to use information related to lipid, etc. Further, as the sensor device for acquiring the biological information, it is possible to use a lipid measurement device, etc.

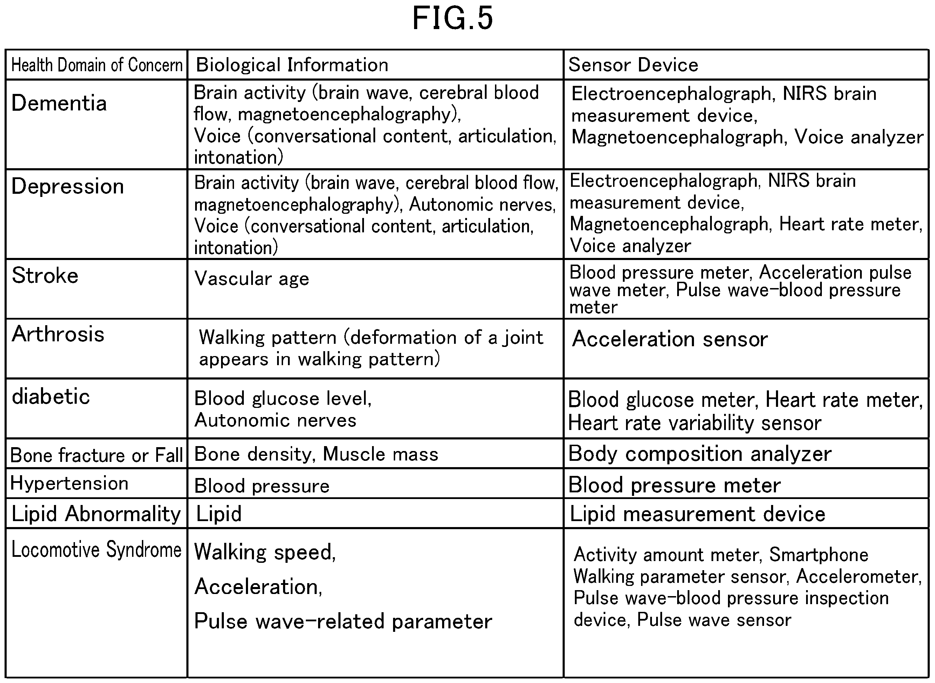

Any of the above sensor devices is a device which has already become usable at home or has a prospect of practical use. These are listed in FIG. 5.

Further, the health domain of concern may be locomotive syndrome. Locomotive syndrome (hereinafter referred to as "LS") is a concept proposed in 2007 by the Japanese Orthopedic Association, and defined as a state in which a disorder of locomotive organs causes a decline in locomotive function. The locomotive function means walking and standing/sitting, and it is considered that as LS progresses, the risk of needing nursing care becomes higher. More specifically, LS follows the following process: while each tissue of bone, cartilage, muscle, etc., constituting locomotive organs, gradually decreases quantitatively and qualitatively with age, an underlying disease such as osteoarthritis pertaining to cartilage, or osteoporosis pertaining to bone becomes latent, whereafter, for some reason, the underlying disease comes to the front (develops), and a symptom such as knee pain, low back pain or bone fracture appears and progresses. Such a symptom is likely to be recovered by detecting it early and taking a preventive measure or by providing a medical treatment even if it progresses to some extent. Thus, it is significant to implement a remediation measure after performing screening or judgment of LS in a reversible stage. The judgment of LS can be performed by a stand-up test, a two-step test, a locomo-25 questionnaire, etc. LS is a disease of muscle, bone or cartilage, classified by such a test, and is relevant to muscle mass, knee osteoarthritis, fall, and bone fracture.

In a case where the health domain of concern is LS, as the biological information, it is possible to use walking speed, acceleration (including vertical acceleration during walking), various pulse wave-related parameters (including pulse wave velocity), etc. Further, as the sensor device for acquiring the biological information, it is possible to use various sensor devices including a wearable terminal. For example, it is possible to use: an activity amount meter, a smartphone, etc., for the walking speed; a walking parameter sensor using an inertial sensor, an accelerometer such as a wireless triaxial accelerometer (including a type capable of being attached to the lumbar spine by a torso belt), etc., for the acceleration; and a pulse wave-blood pressure examination device, a pulse wave sensor (including an optical type), etc., for the various pulse wave-related parameters.

This is also listed in FIG. 5.

The following description will be made on the assumption that the health domain of concern is dementia.

### (Configuration of Terminal Device 100)

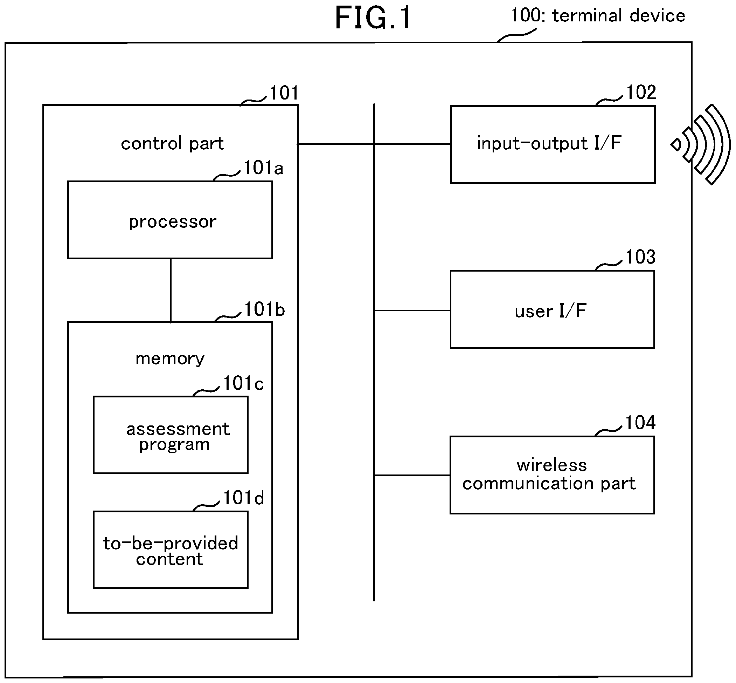

Next, with reference to the drawings, a terminal device 100 as one embodiment of the present invention will be described. Although the terminal device 100 is a terminal unit to be used when a user inputs biological information so as to assess a relevance like a correlation degree and an influence degree of a preventive interventional action, it is also used for allowing the user to input an intervention amount of the preventive interventional action, and derive/calculate the correlation degree and the influence degree of the derived preventive interventional action. The terminal device 100 is typically a mobile computer terminal such as a smartphone. The terminal device 100 may also be any of other types of terminal devices such as a mobile phone, a PDA, and a tablet terminal. FIG. 1 is a block diagram showing the configuration of the terminal device 100. The terminal device 100 comprises a control part 101, an input-output interface (I/F) 102, a user interface (I/F) 103, and a wireless communication part 104. The terminal device 100 is typically a mobile computer terminal such as a smartphone having a function of executing a given program. The control part 101 is a circuit for executing various functions of controlling an operation of the terminal device 100, and is typically a data processing circuit comprising a processor 101a, a temporary memory (not illustrated), and a memory 101b, which are connected to each other via a bus. The memory 101b is typically a non-volatile memory such as a flash ROM, and stores therein an assessment program 101c. In the control part 101, the processor 101a functioning as a CPU is configured to read the assessment program 101c stored in the memory 101b, and execute the assessment program 101c using a work area of the temporary memory such as a RAM, thereby performing an operation for attaining various functions related to relevance assessment. A distinctive function of the terminal device 100 according to the present invention is realized by executing the assessment program 101c such that an execution module appropriate to the function is formed. The memory 101b also stores therein a to-be-provided content 101d. The to-be-provided content 101d is data such as music data or photographic data to be provided for applying a stimulus to the user when detecting a brain wave of the user as the biological information. The to-be-provided content 101d may be a questionnaire, a quiz, a game, or a content consisting of a preventive interventional action having a training element such as brain training. Such data or content is preferably prepared as many types of pieces of data or contents so as to avoid tiresomeness or habituation. In the control part 101, the processor 101a is operable to read and reproduce such a to-be-provided content 101d, and display the to-be-provided content 101d on, e.g., a display of the user I/F 103, etc., thereby providing the to-be-provided content 101d to the user.

The input-output I/F 102 is an interface circuit for transmitting and receiving data with respect to an external sensor, etc., via a near-field communication function, typically Bluetooth (registered trademark), wherein the interface circuit comprises an antenna, a high-frequency circuit, and a data processing circuit. As the external sensor, the after-mentioned wearable sensor 200 is typically used. It should be noted here that the input-output I/F 102 may be a wired interface, and configured such that the external sensor is connected to the terminal device 100 in a wired manner.

The user I/F 103 is a circuit for performing therethrough information exchange with the user. Examples of such a circuit include: a touch panel or buttons for allowing the user to input information such as operational instructions or data, therethrough, or a circuit for driving the touch panel or buttons; and a display, speaker or headphone terminal for outputting, to the user, information such as display data or audio data, therethrough, or a circuit for driving the terminal. The wireless communication part 104 is a circuit for wirelessly performing data communication with an external network such as a mobile telephone network, and allows access to the Internet therethrough. The wireless communication part 104 is typically a circuit comprising an antenna, a high-frequency circuit, and a data processing circuit. Through the wireless communication part 104, the terminal device 100 can access a server for providing a given function, via the Internet.

A wearable sensor 200 is a sensor for acquiring biological information in a state in which it is attached to the body of the user. Although this embodiment will be described by taking a headphone-type brain wave sensor as a specific example, the biological information is not limited to a brain wave as long as it is relevant to the health degree, and the type of wearable sensor may be any other type such as a wristband type, a necklace type or a belly band type as long as it is capable of acquiring a target piece of biological information. The sensor composed of such a wearable sensor imposes less burden on the user in an attached state, so that it is expected to readily acquire biological information, and reduce a burden on the user during long-term monitoring. However, the sensor for acquiring biological information is not limited to the wearable sensor, but may be a commonly-used sensor to be attached to the body during measurement.

### (Operation of Terminal Device 100)

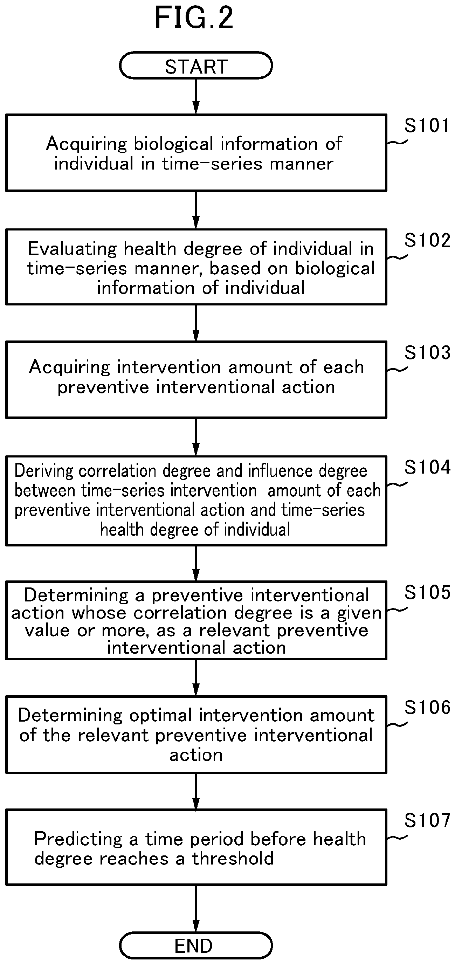

Next, with reference to the drawings, the operation of the terminal device 100 will be described. FIG. 2 is an operation flow of the terminal device 100. A method of the present invention to be implemented in the terminal device 100 is a method for assessing, based on one or more pieces of biological information of an individual/each person each deemed to be relevant to a health degree in a health domain of concern of the individual, a relevance between the health degree and each of one or more preventive interventional actions which are likely to contribute to maintaining or improving the health degree of the individual.

The control part 101 acquires biological information of an individual as a user in a time-series manner (step S101). The biological information is information which is deemed or likely to be relevant to a health degree in a given health domain of concern of the user, and measurement thereof makes it possible to objectively determine the health degree. In order to check a time-series transition indicating how the health degree changes over time, the acquisition of the biological information is made by performing time-series monitoring. The time-series monitoring means performing the acquisition of the biological information periodically with a certain frequency, e.g., one per day, once per week, or once per month. The biological information is preferably information which allows the health degree in the given health domain of concern of the user to be determined from a measurement value thereof obtained under normal conditions. However, it may be acquired under a specific condition that a stimulus is applied to the user. Further, a plural types and times of stimuli may be applied.

This embodiment will be described on the assumption that the health domain of concern of the user is cognitive function, and the biological information is a brain wave as an example. Generally, the degree of cognitive function is assessed by a paper test or an interview with a medical doctor. In the present invention, the degree of cognitive function can be objectively determined from a brain wave as biological information, without performing a paper test or an interview with a medical doctor. This makes it possible to reduce a burden on the user. Preferably, the brain wave is acquired in a state in which a given stimulus is applied to the user. This is because the degree of sensitivity to the given stimulus is considered to appear as the degree of change in brain wave, and the magnitude of the sensitivity is considered to relate closely to the degree of cognitive function. The stimulus may be one type of stimulus based on music or photographic image, and a brain wave is acquired in a state in which such a stimulus is applied to the user. The stimulus is preferable of a type capable of causing a large change in brain wave, wherein differences among individuals in terms of sensitivity to this type of stimulus are small. It is considered that, in a person having normal cognitive function, a large change arises in brain wave in response to such a stimulus, whereas, in a person having deteriorated cognitive function, a change does not so significantly arise in brain wave due to deterioration in sensitivity to a stimulus. Therefore, the degree of cognitive function can be derived by measuring the degree of change in brain wave of a user caused in response to a stimulus applied to the user.

When the number of types of stimuli is increased, differences among individuals in terms of sensitivity to a stimulus are averaged. Thus, it is considered that the accuracy of measurement of cognitive function can be improved by increasing the number of types of stimuli. The following description will be made based on a specific example where a brain wave is acquired in a state in which two types of stimuli are applied to the user. In this specific example, a brain wave of the user is acquired in two states during wakefulness and rest of the user: one state in which a music-based stimulus is applied to the user; and the other state in which a photographic image-based stimulus is applied to the user. There has been known a technique of assessing the state of cognitive function from brain waves acquired in the above two states.

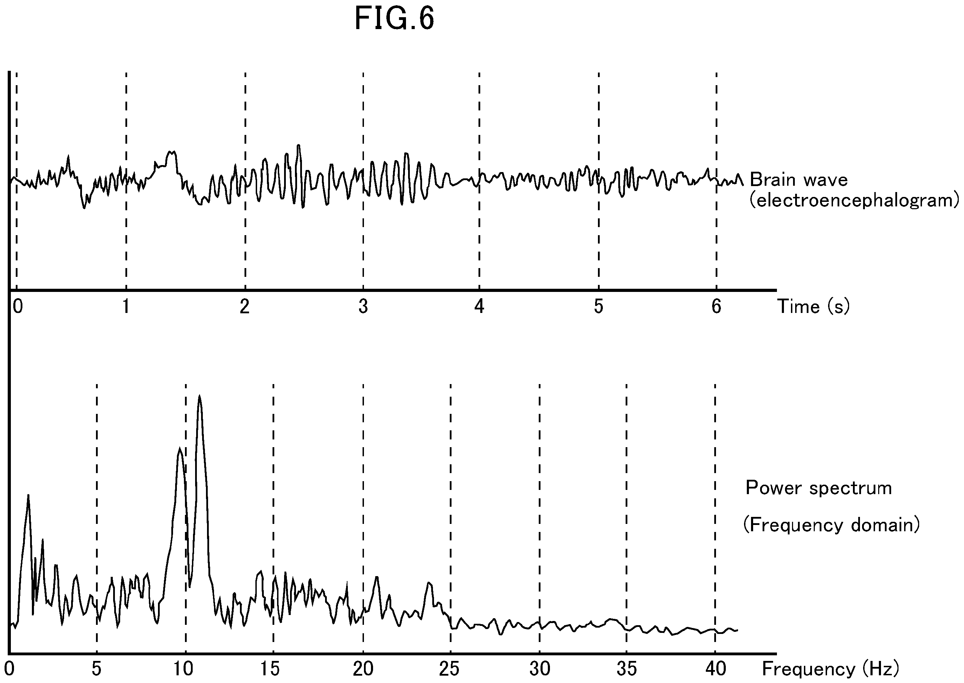

Here, a specific technique of deriving, from biological information, the degree of cognitive function in the case where the health domain of concern is dementia will be described. In a research paper authored by Hiroshi YOSHIMURA, et al., titled "Evaluations of dementia by EEG frequency analysis and psychological examination", the Journal of Physiological Sciences (2010) 60: pp 383-388, 2010, the Physiological Sciences of Japan, it is described that, as a result of acquiring a brain wave in each of a state in which a music-based stimulus is applied to a subject and a state in which a photographic image-based stimulus is applied to the subject, and subjecting the resulting brain waves to frequency analysis, a characteristic pattern was observed in the intensity of a brain wave in a specific frequency band, depending on the degree of dementia. Based on this finding, it is considered that the stage of dementia can be determined from a brain wave which is objective biological information. In this research paper, it is described that, in order to evaluate the stage of dementia, with a focus on EEG (electroencephalogram) rhythms and the HDS-R (revised version of Hasegawa-dementia-rating scale), a brain wave is acquired from each of a person with dementia and a normal person as a control in a state in which each of a music-based stimulus and a photographic image-based stimulus is applied thereto respectively, and subjected to frequency analysis. As a result, with regard to a change between a rhythm pattern of the brain wave (frontal EEG wave) acquired in the state of the music-based stimulus is applied and a rhythm pattern of the brain wave acquired in the state of the photographic image-based stimulus is applied, a large change was observed in the normal person as a control, whereas a change was not so significantly observed in the person with dementia, i.e., a difference was observed between the normal person as a control and the person with dementia. Among various frequency bands of the brain wave, a large difference was observed particularly in an α2 wave range (9 to 11 Hz). FIG. 6 is a chart representing an example of a power spectrum (frequency domain) corresponding to a brain wave. A brain wave having a frequency component of 4 to 7 Hz, and a brain wave having a frequency component of 7 to 9 Hz are referred to respectively as "θ wave" and "α1 wave", and a brain wave having a frequency component of 9 to 11 Hz, and a brain wave having a frequency component of 11 to 13 Hz are referred to respectively as "α2 wave" and "α3 wave". Further, a brain wave having a frequency component of 13 to 30 Hz is referred to as "β wave". A brain wave is shown in the form of waveform on the upper side of FIG. 6 in which the horizontal axis represents time, and a power spectrum (frequency domain) obtained by converting the brain wave to a frequency domain is shown in the form of intensity distribution of frequency components on the lower side of FIG. 6 in which the horizontal axis represents frequency. The example in FIG. 6 shows that the intensity of the α2 wave around 10 Hz is greater than that of other frequency components.

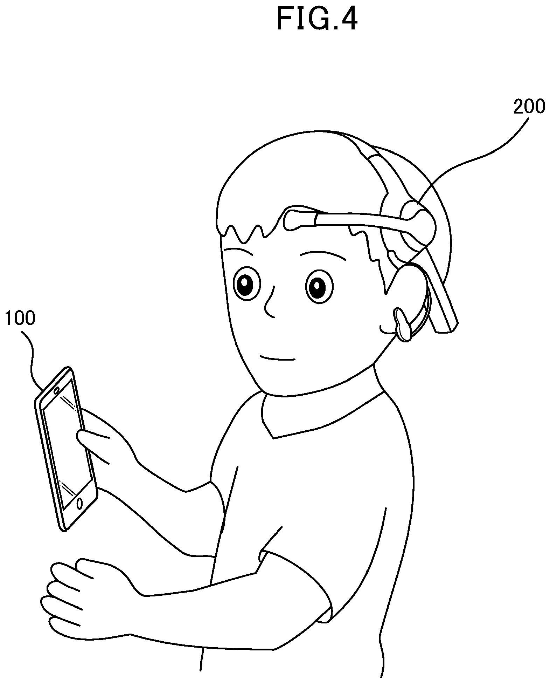

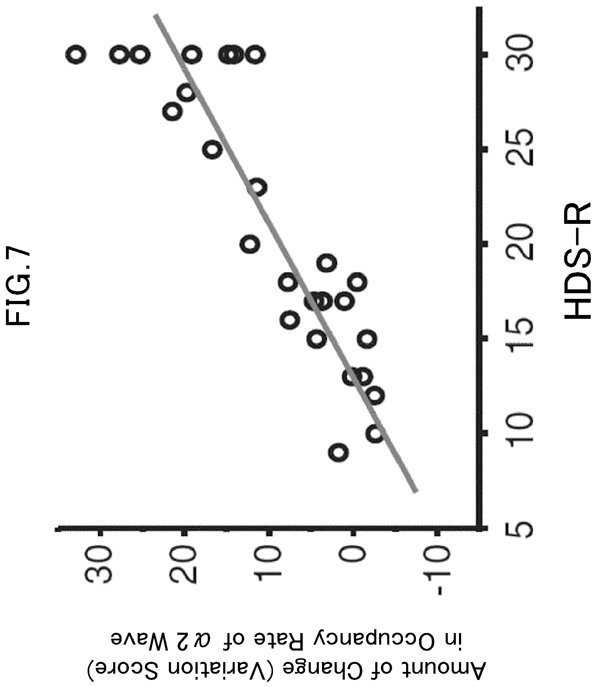

In order to quantify variability of a rhythm pattern of each of various frequency bands, the amount of change in occupancy rate of the α2 wave was derived by subtracting the occupancy rate of the α2 wave in the case of applying the photographic image-based stimulus from the occupancy rate of the α2 wave in the case of applying the music-based stimulus, and assessment was performed using the derived change amount. As a result, it has been found that, particularly in the α2 wave, the amount of change in occupancy rate thereof decreases along with a decrease in the HDS-R. Here, the occupancy rate of the α2 wave means a value derived by [the intensity of the α2 wave / the sum of the intensities of the θ wave, the α1 wave, the α2 wave, the α3 wave, and the β wave]. FIG. 7 is a graph cited from the graph of Alpha 2 in Fig. 4 of the research paper authored by Hiroshi YOSHIMURA, et al., wherein the graph shows the relationship between the HDS-R and the amount of change in occupancy rate of the α2 wave. From FIG. 7, it is able to understand that there is an approximately straight-line relationship between the HDS-R and the change amount (variation score), and, when the HDS-R and the amount of the change are denoted, respectively, by x and y, it is derived that the straight-line approximately satisfies the following relational formula: y = 1.225x - 15.95, because it passes through two points (10, - 3.7) and (30, 20.8). In this above research paper, the occupancy rate is calculated as an average value for 180 seconds under conditions including a sampling rate of 1 second, and a measurement time period of 180 seconds.

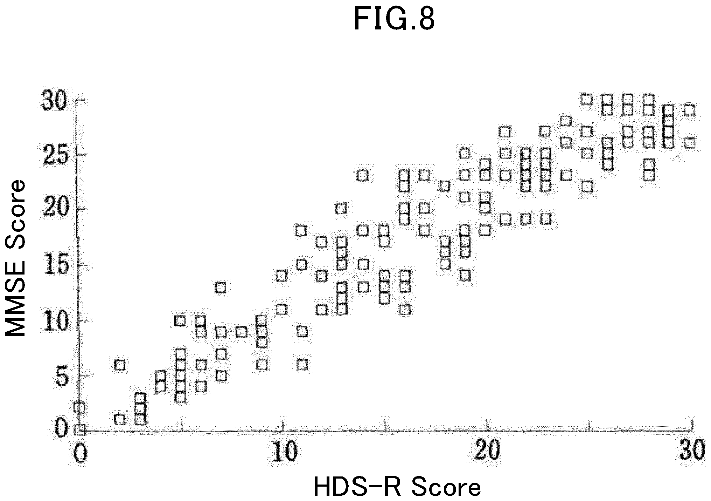

Here, an HDS-R score is extremely strongly correlated with an MMSE (Mini Mental State Examination) score which is used as a general index of cognitive function. FIG. 8 is a graph cited from FIG. 2 of a research paper authored by Shinji KATO, et al., titled "Development of the revised version of Hasegawa's Dementia Scale (HDS-R)", the Journal of Geriatric Psychiatry, Vol. 2, No. 11, Nov. 1999, Japanese Psychogeriatric Society, wherein the graph shows the relationship between the HDS-R score and the MMSE score. From FIG. 8, it is able to understand that there is a proportional relationship with a proportional constant of about 1 between the HDS-R score and the MMSE score, i.e., there is the following relationship: HDS-R score ≒ MMSE score. Considering the results in FIGS. 7 and 8 together, it can be said that there is a straight-line relationship between the amount of change in the brain wave and the MMSE score as with the case of the HDS-R, and thus it is possible to derive the MMSE score from the amount of change in the brain wave. In this embodiment, the amount in change in occupancy rate of the α2 wave is derived by subtracting the occupancy rate of the α2 wave in the case of applying the photographic image-based stimulus from the occupancy rate of the α2 wave in the case of applying the music-based stimulus, and the MMSE score is derived by assigning the derived amount to the relational formula: y = 1.225x - 15.95, where x denotes the MMSE score, and y denotes the amount of change in occupancy rate of the α2 wave. This MMSE score serves as the health degree derived using a brain wave as biological information in the case where the health domain of concern is cognitive function.

The following description will be made about a case where the health domain of concern is locomotive syndrome. In a case where the biological information is the walking speed, it is known that the probability of onset becomes higher as the walking speed becomes lower. In a case where the biological information is the vertical acceleration during walking, it is known that, in gait variability based on assessment of an autocorrelation coefficient on a vertical axis measured using an accelerometer, the probability of onset becomes higher as the autocorrelation coefficient becomes smaller. In a case where the biological information is any one of the various pulse wave-related parameters (including the pulse wave velocity), it is known that the probability of onset becomes higher, for example, as the pulse wave velocity becomes higher. Specific examples will be described below.

With regard to the relationship between the walking speed and locomotive syndrome, in an article authored by Kim Hunkyung, et al., titled "Old-age locomotion capability and LS", LOCO CURE vol. 3, No. 2 (2017), pp 46-51 (138-143), 2017, Sentan Igaku-Sha Ltd., it is shown that there is a correlation between the waking speed and each of the muscle mass, knee osteoarthritis, previous bone fracture, and fall. Table 1 of the article shows that, whether the walking speed is less than 0.8 m/s, or equal to or greater than 0.8 m/s has a significant correlative relationship having a p-value of 0.001 to 0.034, with regard to the muscle mass of the entire body, the muscle mass of the leg, the presence or absence of knee osteoarthritis, the presence or absence of a previous history of bone fracture after age 60, and the presence or absence of a fear of fall. Further, Table 2 of the article shows that, when the walking speed and knee pain are expressed, respectively, as an independent variable and a dependent variable, there is a significant correlation having: an odds ratio of 0.979; a 95% confidence interval of 0.968 to 0.991; and a p-value of 0.001, and, when the walking speed and fall are expressed, respectively, as an independent variable and a dependent variable, there is a significant correlation having: an odds ratio of 0.976; a 95% confidence interval of 0.954 to 0.997; and a p-value of 0.029

With regard to the relationship between the walking speed and locomotive syndrome, in an article authored by S. MURAKI, et al., titled "Physical performance, bone and joint diseases, and incidence of falls in Japanese men and women: a longitudinal cohort study", Osteoporos International (2013) 24: pp 459-466, 2013, the International Osteoporosis Foundation and National Osteoporosis Foundation, Table 2 shows that there is a significant correlation having a p-value of less than 0.001, between whether or not a person is likely to fall and the walking speed.

Further, with regard to the relationship between gait variability based on the autocorrelation coefficient of acceleration on the vertical axis and locomotive syndrome, in a research paper authored by Hiromi MATSUMOTO, et al., titled "Gait variability analysed using an accelerometer is associated with locomotive syndrome among the general elderly population: The GAINA study", Journal of Orthopaedic Science 21 (2016), pp 354-360, 2016, the Japanese Orthopaedic Association, Table 4 shows a result of verification of the presence or absence of a correlation between gait variability based on acceleration on the vertical axis during walking (AC-VT Step) and locomotive syndrome, with 224 subjects, and Table 5 shows that, in regression analysis of the relationship between gait variability based on the acceleration on the vertical axis during walking and locomotive syndrome, there is a significant correlation having an adjusted odds ratio of 0.943; a 95% confidence interval of 0.892 to 0.996; and a p-value of 0.037.

As above, the presence or absence of locomotive syndrome is correlated with the waking speed or the acceleration on the vertical axis. Thus, the health degree in the case where the health domain of concern is locomotive syndrome (i.e., an index of the degree of progression of locomotive syndrome, such as a LS degree) can be derived, using the walking speed or the acceleration on the vertical axis as biological information obtainable from the living body.

With regard to the relationship between each of the various pulse wave-related parameters and locomotive syndrome, in a research paper authored by Tomonori SATO, et al, titled "Relationship between Brachial-Ankle Pulse Wave Velocity and Pre-sarcopenia in Hypertensive Men", the journal of Japan Society of Clinical Pathology, Vol. 45, No. 5, pp 133-140, 2015, Japan Society of Clinical Pathology, there is shown a result of study on the relationship between pre-sarcopenia syndrome and progression of arterial stiffness, based on measurement of ba PWV (brachial-ankle Pulse Wave Velocity) which is a sort of the pulse wave velocity (PWV), i.e., a velocity at which an arterial pulse wave generated by blood ejection from the heart is transmitted to the periphery, and skeletal muscle mass. Here, the term "sarcopenia" is one element of locomotive syndrome, and means declining of muscle strength or physical functions, due to a decrease in the muscle mass mainly by aging. A decrease in the skeletal muscle mass means progression of sarcopenia. Further, a general relationship is known that as arterial stiffness progresses, the pulse wave velocity becomes higher. Further, in various reports of researchers or the like, it is reported that there is a negative correlation between the ba PWV and the skeletal muscle mass (as the ba PWV becomes higher, the skeletal muscle mass becomes smaller. Further, the study result shows that, in multiple logistic regression analysis using "a cutoff point of a ba PWV of 1800 cm/sec" as an objective, variable, pre-sarcopenia syndrome was an explanatory factor independent of classical factors such as age, blood pressure, pulse, and blood glucose. As above, it is shown that there is a correlative relationship in which, as the brachial-ankle pulse wave velocity (ba PWV) becomes higher, the skeletal muscle mass becomes smaller, and sarcopenia further progresses. Here, the further progression of sarcopenia is understood to be synonymous with further progression of locomotive syndrome. It is also understood that there is a similar relationship in the pulse wave velocity (PWV), as well as the brachial-ankle pulse wave velocity (ba PWV). Further, even when using any one of the various pulse wave-related parameters to be acquired in a common wave examination, it is considered to be correlated with the degree of progression of arterial stiffness, thereby allowing the degree of locomotive syndrome to be determined in a similar manner.

As mentioned above, the degree of progression of locomotive syndrome is correlated with any of the various pulse wave-related parameters. Thus, the health degree in the case where the health domain of concern is locomotive syndrome (i.e., an index of the degree of progression of locomotive syndrome, such as the LS degree) can be derived, using the pulse wave-related parameter such as the pulse wave velocity as biological information obtainable from the living body.

Returning to the case where the health domain of concern is dementia, the rest of the related description will be continued below.

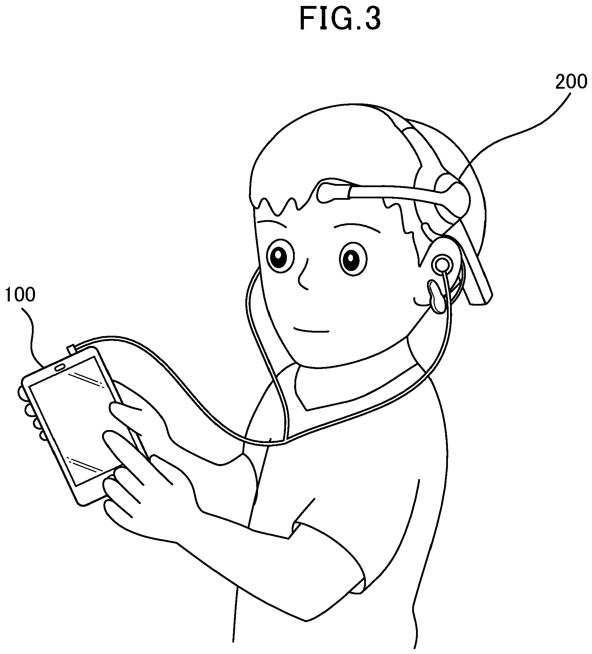

The biological information of the user is picked up, preferably, by a wearable terminal attached to the body of the user. In this embodiment, a wearable sensor 200 which is a headphone-type brain wave sensor is used. Further, the music-based or photographic image-based stimulus is provided such that the to-be-provided content 101d reproduced by the control part 101 of the terminal device 100 is output as music from a speaker or a headphone terminal of the terminal device 100, or is displayed as a photographic image on a display of the terminal device 100. FIG. 3 is a conceptual diagram of a scene where a brain wave is acquired in a state in which music is provided to the user. The user manipulates the terminal device 100 to operate a relevance assessment application. Preferably, at that time, a guidance is provided to the user by voice or text so as to instruct the user to listen to music for a while with the wearable sensor 200 on. The user wears the wearable sensor 200 like a headphone, in a state in which two electrodes are brought into contact with the forehead and the ear lobe, respectively. According to the operation of the application, the control part 101 reproduces the to-be-provided content 101d of music and provides the music to the user. During that time, the wearable sensor 200 acquires a brain wave from the electrodes, and a sensor circuit of the wearable sensor 200 transmits a signal indicative of the brain wave to the input-output I/F 102 of the terminal device 100. The signal indicative of the brain wave is typically a signal obtained by subjecting the brain wave to A-D conversion by sampling.

When the acquisition of a brain wave in the state in which the music is provided to the user is completed, the control part 101 stops the reproduction of the music, and then restarts to acquire a brain wave in this state while displaying a photographic image on the display, according to the operation of the relevance assessment application. FIG. 4 is a conceptual diagram of a scene where a brain wave is acquired in a state in which a photographic image is provided to the user. Preferably, in this process, a guidance is provided to the user by voice or text so as to instruct the user to look at the photographic image for a while with the wearable sensor 200 on. According to the operation of the application, the control part 101 displays the to-be-provided content 101d of a photographic image and provides the photographic image to the user. During that time, the wearable sensor 200 acquires a brain wave from the electrodes, and the sensor circuit thereof transmits a signal indicative of the brain wave to the input-output I/F 102 of the terminal device 100. When the acquisition of a brain wave in the state in which the photographic imager is provided to the user is completed, the control part 101 stops the displaying of the photographic image, according to the operation of the relevance assessment application. In this way, biological information (brain waves acquired under application of the two types of stimuli) of the user is obtained. Then, the acquisition of such biological information is periodically performed, so that the biological information will be acquired as time-series data. The biological data acquired so far is preferably stored in the memory 101b in the form of file or the like.

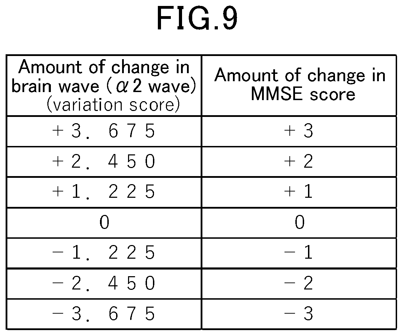

Subsequently, based on the acquired biological information, the control part 101 evaluates the health degree of the individual in a time-series manner (step S102). Specifically, according to the operation of the relevance assessment application, the control part 101 concerts the brain waves acquired under application of the two types of stimuli to a frequency domain by using an algorithm such as FFT algorithm to derive at least the intensity of the α2 wave, and the sum of the intensities of the θ wave, the a1 wave, the α2 wave, the α3 wave and the β wave (hereinafter referred to as "the θ to β waves"), and derives the occupancy rate the α2 wave, i.e., a rate of the intensity of the α2 wave to the sum of the intensities of the θ to β waves", for each of the brain waves acquired under application of the two types of stimuli. Then, the control part 101 subtracts the occupancy rate the α2 wave in the case of applying the photographic image-based stimulus from the occupancy rate the α2 wave in the case of applying the music-based stimulus to calculate the amount of change in occupancy rate the α2 wave. Further, the control part 101 assigns the calculated change amount to the relational formula: y = 1.225x - 15.95, where x denotes the HDS-R score, and y denotes the amount of change in occupancy rate the α2 wave, thereby deriving the MMSE score. FIG. 9 is a table representing the relationship between the amount of change in occupancy rate of the α2 wave and the amount of change in the MMSE score. This MMSE score serves as the health degree derived using the brain waves acquired under application of the two types of stimuli, as biological information, in the case where the health domain of concern is cognitive function. In this way, assessment of the health degree (MMSE score) of the user is performed. This assessment is periodically performed, so that the health degree will be assessed in a time-series manner. The health degrees acquired and calculated so far are preferably stored in the memory 101b.

In the steps S101 and S102, what is important is to: acquire, as biological information, brain waves under application of two types of stimuli to derive the occupancy rate of the α2 wave for each of the brain waves; derive the amount of change in occupancy rate of the α2 wave; and calculate the health degree corresponding to the derived change amount. That is, it is understood that the two types of stimuli stimulate the brain differently, thereby exerting different influences on the intensities of frequency components (θ to β waves) of a brain wave. Based on this understanding, it is considered that the magnitude of brain response to a change in stimulus can be quantified with a high degree of accuracy by selecting a frequency component (including a frequency component other than the α2 wave) of a brain wave whose intensity change has a strong correlation with the two types of stimuli, irrespective of contents of the two types, and deriving the amount of change in the selected frequency component. Here, it is inferred that a person having deteriorated cognitive function deteriorates in terms of sensitivity to a stimulus, and thus a change in brain response to a change in type of stimulus is small, resulting in a small amount of change in a brain wave. Thus, it is inferred that the health degree corresponding to cognitive function can also be derived from the amount of change in occupancy rate between specific frequency components of two types of brain waves acquired under application of two types of stimuli other than music and photographic image. That is, it is inferred that, in either case, a tendency appears that, as the change amount becomes smaller, the health degree corresponding to cognitive function becomes lower. From these viewpoints, the two type of stimuli may be any combination of various stimuli, such as sound, music, printed image, photographic image, video image, questionnaire, quiz, game and brain training. A correspondence relationship between the change amount and the health degree (MMSE score) obtained in the above step may be experimentally derived according to the two types of stimuli used. The MMSE score can be derived based on this correspondence relationship and using the change amount as an index. Here, the two types of stimuli need not necessarily be different in terms of type. For example, they may be two brain trainings which are different in terms of content. Further, one of the two types of stimuli may be a stimulation-free condition in which no specific stimulus is applied, during wakefulness and rest, or during sleep. A stimulation operation of applying an intellectual stimulus such as questionnaire, quiz, game and brain training is regarded itself as the preventive interventional action.

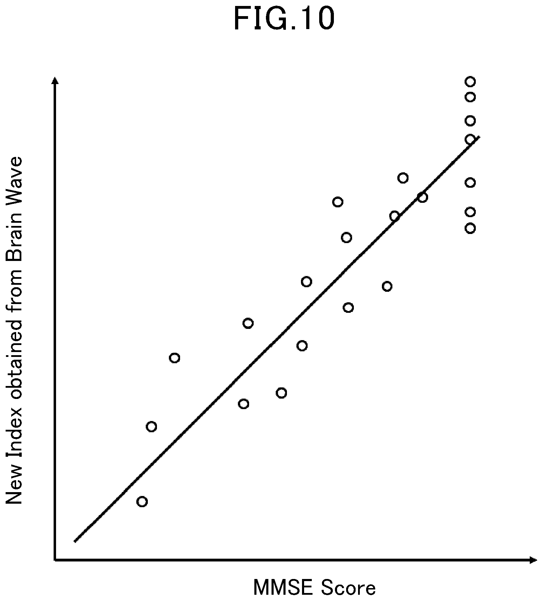

Another brain wave-related index correlated with the MMSE score may be experimentally found. In this case, the MMSE score can be derived by quantifying a correspondence relationship between this index and the MMSE score, and analyzing an acquired brain wave using the index. FIG. 10 is a graph representing an example of a brain wave-related index having a significant relationship with the MMSE score. In FIG. 10, specific values (indicated by ○) of the experimentally obtained index and the MMSE score are plotted, wherein there is a proportional relationship as indicated by a straight line between the index and the MMSE score. As above, such an experimentally-found index can also be used to derive the MMSE score.

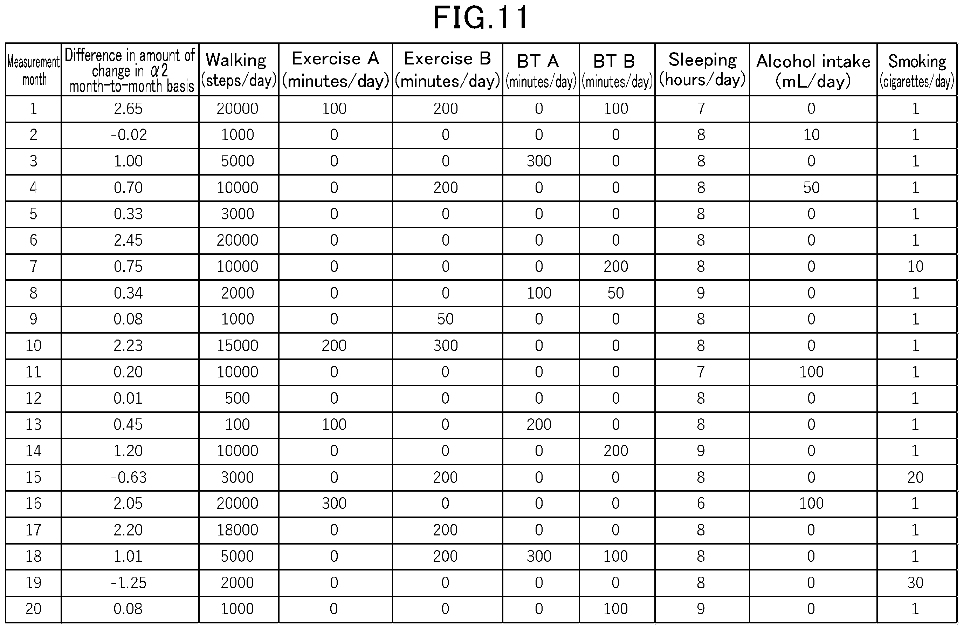

Subsequently, the control part 101 acquires an intervention amount of each of one or more preventive interventional actions in a time-series manner (step S103). Specifically, according to the operation of the relevance assessment application, the control part 101 accepts an input of the intervention amount of each of the one or more preventive interventional actions from the user. Preferably, the intervention amount is an activity amount of a given activity of the user. The control part 101 is configured to preliminarily set how to input the type and the intervention amount of each preventive interventional action to be used. For example, in a case where the activity amount such as the number of steps and a sleep time period is measured by a device such as a wearable device, the control part 101 accepts an input of the intervention amount and acquires the intervention amount through communication with the device via the input-output I/F 102. In a case where the intervention amount is that of a preventive interventional action provided to the user by the terminal device 100, so as to apply an intellectual stimulus such as questionnaire, quiz, game and brain training, the control part 101 stores the amount (time period) of the preventive interventional action provided, a result (achievement) thereof, etc., and acquires it as the intervention amount. In a case where the intervention amount is a numerical valve relevant to user's daily life, such as the amount of alcohol intake, and the number of cigarettes smoked, and needs to be input by a user himself/herself, the control part 101 displays an instruction for inputting one or more preventive interventional actions therefor on, e.g., a display of the user I/F 103, to prompt the user to input an intervention amount of each of the one or more preventive interventional actions via, e.g., a touch panel of the user I/F 103. FIG. 11 is a table representing examples of a preventive interventional action and intervention amounts thereof acquired in a time-series manner. As the preventive interventional action, the table in FIG. 11 shows: walking; exercise A; exercise B; brain training A (BT A); brain training A (BT B); sleeping; alcohol intake; and smoking, wherein the intervention amount of each preventive interventional action and biological information are acquired or input monthly over a measurement period of 20 months. The biological information is presented in the column "Difference in amount of change in α2 on month-to-month basis" as the amount of month-to-month change in the α2 wave in each brain wave acquired under application of two types of stimuli. As the intervention amount for each preventive interventional action, the table shows: the number of steps for the walking; an exercise time period for the exercise A; an exercise time period for the exercise B; an implementation time period for the brain training A (BT A); an implementation time period for the brain training A (BT B); a sleep time period for the sleeping; the amount of alcohol intake for the alcohol intake; and the number of cigarettes smoked for the smoking.

As the intervention amount of the preventive interventional action, it is possible to use any of various intervention amounts, e.g., the following intervention amounts:
(i) the amount of walking;
(ii) an exercise amount of a given exercise;
(iii) the amount of implementation of a given cognitive training;
(iv) a sleep time period;
(v) the amount of nutrition intake;
(vi) the amount of medication/supplement/health food intake;
(vii) the amount of auditory stimulation by a given sound or music piece;
(viii) the amount of visual stimulation by a given still or moving image:
(ix) the amount of electrical or magnetic stimulation to the brain;
(x) the amount of alcohol intake; and
(xi) the number of cigarettes smoked.

With regard to each of the preventive interventional actions corresponding to (i) to (ix), a larger amount thereof before reaching a moderate amount is generally considered to be more desirable. In this case, assuming that the amount of each of these actions is determined as the intervention amount, it can be considered that, as the intervention amount becomes larger, the health degree is further improved. On the other hand, with regard to each of the preventive interventional actions corresponding to (x) to (xi), a smaller amount thereof is generally considered to be more desirable. For example, in a case where alcohol intake or smoking becomes a habit, reducing the amount of alcohol intake or the number of cigarettes smoked, from a habitual amount or number, becomes a preventive interventional action. In this case, assuming that the amount of alcohol intake or the number of cigarettes smoked is determined as the intervention amount, it can be considered that, as the intervention amount becomes smaller, the health degree is further improved.

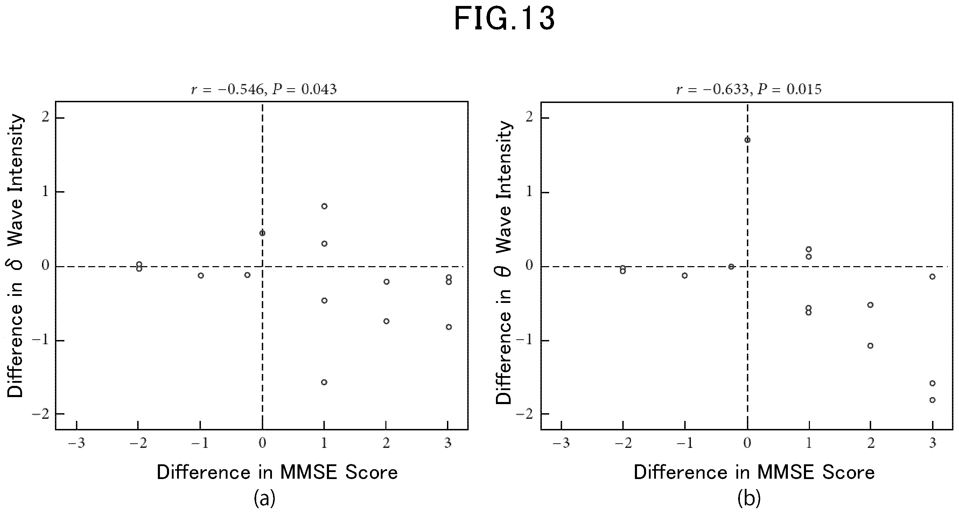

As a document disclosing a preventive interventional action exerting an influence on cognitive function, there are the following documents. In a research paper authored by Charis Styliadis, et al, titled "Neuroplastic Effects of Combined Computerized Physical and Cognitive Training in Elderly Individuals at Risk for Dementia: An eLORETA Controlled Study on Resting States", Neural Plasticity, Volume 2015, Article ID 172192, there is shown a result of research about what kind of influence the effect of a combined cognitive and physical training exerts on cortical activity measured by electroencephalogram (EEG). In the research, 70 patients with mild cognitive impairment (MCI) were divided into three intervened groups and two control groups, and they are compared with each other. Here, EEG (brain wave at rest for 5 minutes) was measured before and after the intervention. Cortical EEG sources were modelled by exact low resolution brain electromagnetic tomography (eLORETA). Cognitive function was measured before and after the intervention by tests including MMSE (minimental state examination). Here, each of physical training and cognitive training was implemented for 5 hours/week over 8 weeks. As a result, a significant improvement was observed in the combined physical and cognitive training, and, as an observation about brain waves, a reduction in δ, θ and β waves was observed. FIG. 13 illustrates graphs cited from FIGURE 3 of the research paper authored by Charis Styliadis, et al., wherein FIG. 13(a) shows the relationship between a difference in the MMSE score and a difference in δ wave intensity (current density reconstructions (C DR)), and FIG. 13(b) shows the relationship between a difference in the MMSE score and a difference in θ wave intensity. FIG. 13 shows that, before and after intervention of the combined physical and cognitive training, the MMSE score increases in 9 examples, remains unchanged in 1 example, and decreases in 4 examples. Further, FIG. 13 shows a tendency that each of the δ wave and θ wave intensities gradually decreases along with an increase in the MMSE score. This shows that a brain wave can serve as an index of a non-pharmacological intervention in mild cognitive impairment. The above research paper authored by Charis Styliadis, et al., is understood to show that: the combined physical and cognitive training is effective in improving cognitive function (MMSE score); the degree of the effect varies between individuals; and there is a relationship between the degree of cognitive function and the intensity of a brain wave. From the viewpoint of the presence of a difference in the effect of the combined physical and cognitive training among individuals, it is understood that there is an important meaning in selecting a preventive interventional action correlated with each individual. Further, although being different from the technique in the research paper authored by Hiroshi YOSHIMURA, et al., it is also understood that the degree of cognitive function can be known from a brain wave.

In the case where the health domain of concern of the user is locomotive syndrome, it is known that examples of a usable intervention method include: exercise (including locomotion training and aerobic exercise); a heating sheet; food intake, exposure to ultraviolet light; a portable electrical muscle stimulation device such as SIXPAD (registered trademark), and a social activity-related method

In this case, as the intervention amount of the preventive interventional action, it is possible to use: the amount of exercise (including the amount of aerobic exercise); the amount of usage of a heating sheet; the amount of locomotion training (muscle training such as squat, single-leg standing in an eye-opened state, heel raise, forward lunge, and dumbbell exercise; the amount of nutrient intake (the adequacy degree of energy intake, a food intake diversity score, etc.); the amount of usage of a portable electrical muscle stimulation device; the amount of participation in social activity; the number of steps; a movement distance; the duration of exposure ultraviolet light; an eating time period; the amount of calorie intake; the amount of intake of a specific nutrient; the amount of sleep (including a sleep time period (which may be a non-REM sleep time period), a ratio between a REM sleep time period and a non-REM sleep time period, etc.); the amount of consumed calorie; and a pulse rate. Specific examples will be shown below.

In an article authored by YAMADA, et al., titled "Findings from longitudinal elderly locomotive organs examinations related to acquisition of exercise habits and improvement in LS degree - LS degree can be improved by aerobic exercise -", Journal of Spine Research, Vol. 8, No. 3, 2017, the Japanese Society for Spine Surgery and Related Research, it is reported that there was a tendency that the LS degree was improved in both male and female by newly starting an aerobic exercise. The aerobic exercise means a mild or moderate load exercise which can be continued for a long time period so as to obtain hemoglobin by aerobic metabolism, and examples thereof include walking, jogging, running, bike riding, aerobics, swimming, and aquabics.

Thus, assuming that the aerobic exercise is determined as the preventive interventional action, and the amount of the aerobic exercise is determined as the intervention amount, it can be considered that, as the intervention amount becomes larger or becomes closer to a proper amount, the health degree is further improved. In this case, the terminal device 100 can acquire the exercise amount of the aerobic exercise as the intervention amount, by communicating with an activity amount meter (which may be a wearable terminal) which has measured the exercise amount of the aerobic exercise, via the input-output I/F 102 in a wireless or wired manner. Alternatively, the terminal device 100 may be configured to accept an input of the exercise amount of the aerobic exercise from the user via the user I/F 103.

In an article authored by Hideaki ISHIBASHI, titled "Exercise therapy and nutritional guidance as LS measures", THE BONE, Vol. 31, No. 3, autumn, 2017, pp 89-94 (325-330), 2017, Medical Review Co., Ltd., the following is shown with regard to the relationship among exercise, food intake and locomotive syndrome.

Firstly, with regard to exercise, locomotion training (hereinafter referred to as "LT") is recommended as an exercise therapy against LS, and consists of four types of exercises: squat, single-leg standing in an eye-opened state, heel raise, and forward lunge. This article reports that assessment values of a locomotion check, a LS degree test, etc., as indexes of LS, were improved by intervention of LT. Specifically, it reports that, as a result of applying a LT-based 6-month intervention to an elderly person which corresponds to one or more items of concern of the locomotion check, the duration of the single-leg standing and a result of a functional reach test were significantly improved, and the number of items of concern of the locomotion check significantly increases. The article also shows that the author and other staffs applied a LT-based 2-month intervention (squat, single-leg standing in an eye-opened state, and heel raise) to 218 elderly persons, and thereby the duration of the single-leg standing, a 10m walking time period, a result of a 3m Timed Up-and-Go Test, and a toe gripping strength were significantly improved. Further, the article reports that, as a result of subjecting 28 elderly men and women who correspond to one or more items of concern of the locomotion check, to implementation of LT and walking for 3 months or 6 months, the duration of the single-leg standing, knee flexion muscle strength, and the number of items of concern of the locomotion check were significantly improved, and anxiety about falls was alleviated. Further, the article reports that, as a result of subjecting 31 community-dwelling elderly persons to implementation of LT or dumbbell exercise for 6 months, a significant improvement was observed in the maximum walking speed, a 30-second chair stand test, the 3m Timed Up-and-Go Test, the number of items of concern of the locomotion check, etc.

In an official site of locomotive syndrome prevention awareness, authorized by the Japanese Orthopaedic Association, the following LTs 1 to 4 are recommended.
1. One-leg standing: improvement in balance ability. Guide for daily exercise: 1 minute for each leg, 3 times/day
2. Squat: lower-limb muscle strength. Guide for daily exercise: repeating the squat 5 to 6 times at the pace of taking a deep breath, 3 times/day
3. Heel raise : calf muscle strength. Guide for daily exercise: 10 to 20 times/set × 2 to 3 sets/day
4. Forward lunge: flexibility of lower limb, balance ability, and muscle strength. Guide for daily exercise: 10 to 20 times/set × 2 to 3 sets/day

Thus, assuming that the locomotion training such as squat, single-leg standing in an eye-opened state, heel raise, forward lunge, or dumbbell exercise is determined as the preventive interventional action, and the amount of the locomotion training is determined as the intervention amount, it can be considered that, as the intervention amount becomes larger, or becomes closer to a proper amount, the health degree is further improved. In this case, the terminal device 100 can acquire the exercise amount of the locomotion training as the intervention amount, by communicating with an activity amount meter (which may be a wearable terminal) which has measured the exercise amount of the locomotion training, via the input-output I/F 102 in a wireless or wired manner. Alternatively, the terminal device 100 may be configured to accept an input of the exercise amount of the locomotion training from the user via the user I/F 103.

Secondly, with regard to food intake, in the article "Exercise therapy and nutritional guidance as LS measures", it is described that it is recommended to maintain the adequate amount of food intake, and take nutrients in a well-balanced manner, and it is reported that a significant relationship is observed between a food intake diversity score obtained by expressing as score, diversity of foods ingested per day, to assess how many types of foods among 10 types of foods are roughly taken per day, and each of a grasping power and a normal walking speed, wherein as the diversity score becomes higher, each of the grasping power and the normal walking speed becomes higher, and the loss of motor function after 4 years becomes smaller.

Thus, assuming the food intake is determined as the preventive interventional action, and the amount of the food intake is determined as the intervention amount, it can be considered that, as the intervention amount becomes closer to a proper amount, the health degree is further improved. Alternatively, assuming the food intake is determined as the preventive interventional action, and the diversity score of the food intake is determined as the intervention amount, it can be considered that, as the intervention amount becomes larger, the health degree is further improved. In this case, the terminal device 100 can accept an input of the food intake amount or the diversity score as the intervention amount from the user via the user interface 103. The diversity score is calculated from an input of the number of types of ingested foods.

In the aforementioned article "Old-age locomotion capability and LS", it is reported that, as a result of verifying respective effects of exercise guidance and heating sheet guidance, by selecting 150 persons with pain in knee as participants in therapeutic intervention and dividing them into an exercise + heating sheet group of 38 persons, an exercise group of 37 persons, an heating sheet group of 38 persons, and a control grope of 37 persons, after a 3-month intervention, with regard to the normal walking speed, a significant improvement was observed in the exercise group and the exercise + heating sheet group, but no significant change was observed in the control group and the heating sheet group, whereas, with regard to stride, a significant increase was observed in he exercise group and the exercise + heating sheet group, which proved that the increase in stride largely contributed to improvement in walking speed of a person with pain in knee.

Thus, assuming that the exercise or the heating sheet is determined as the preventive interventional action, and the amount of the exercise or the amount of usage of the heating sheet is determined as the intervention amount, it can be considered that, as the intervention amount becomes larger, or becomes closer to a proper amount, the health degree is further improved. In this case, the terminal device 100 can accept an input of the exercise amount or the amount of usage of the heating sheet as the intervention amount from the user via the user interface 103.

In an article authored by Seiji YASUMURA, et al., titled "7th Measures against locomotive syndrome - effectiveness of locomocol call", Journal of Orthopedic Surgery, Vol. 64, No. 13 (2013-12), pp 1412-1415, 2013, Nankodo Co., Ltd., it is shown that, in connection with intervention of home exercise as part of locomotion training, making an encouragement call (locomocol call) one to several times per week could raise an intervention method continuation rate and significantly improve the duration of single-leg standing in an eye-opened state.

Thus, assuming that the locomocol call is determined as an indirect preventive interventional action, and the frequency of the locomocol call is determined as the intervention amount, it can be considered that, as the intervention amount becomes larger, the health degree is further improved. In this case, a service provider who makes the locomocol call inputs the frequency (e.g., times/day) of the locomocol call as the intervention amount, to the terminal device 100 via the user I/F 103, the input-output I/F 102, the wireless communication part 104, or the like. In the case of using the user I/F 103, when visiting user's home, the service provider can input the frequency of the locomocol call to the terminal device 100, using a touch panel or the like. Alternatively, the user may input the frequency of the locomocol call to the terminal device 100, using a touch panel or the like, by himself/herself. In the case of using the input/output I/F 102, when the service provider visits user's home with a mobile terminal to which the frequency of the locomocol call is input, the terminal device 100 can acquire the frequency of the locomocol call by communicating with the mobile terminal via the input/output I/F 102, e.g., in a wireless manner. In the case of using the wireless communication part 104, the service provider can transmit the frequency of the locomocol call via the Internet to allow the terminal device 100 of the user to acquire the frequency of the locomocol call.

In a research paper authored by Hideaki ISHIBASHI, et al, titled "Accumulation of verified data about locomotive syndrome, motor function predictability based on elderly locomotion check, and verification of motor function enhancing effect of locomotion training", Musculoskeletal Rehabilitation (2187-8420), Vol. 24, No. 1, pp 77-81 (2013.05), 2013, Japanese Society for Musculoskeletal Medicine, the Abstract shows a result of: subjecting 229 elderly persons aged 65 or older (30 men, 199 women, average age: 76.6) to the locomotion check (LC); guiding a person determined to have locomotive syndrome (LS) on the locomotion training (LT); and verifying the motor function enhancing effect, wherein, in the LC for 218 persons from which checking items could be validly acquired in the initial stage, 56.8% of the 218 persons was determined as a LS group, and the Background shows that: as compared with a non-LS group, the LS group was older, significantly large in terms of the number of falls in the last 6 months, and significantly small in terms of EQ-5D utility value; in all the checking items, motor function was significantly good in the non-LS group; after performing a group guidance for LT, 138 persons (60.3%) performed self-training for 2 months, and thereby the single-leg standing, a result of the functional reach test, the 10m walking time period, the 3m Timed Up-and-Go Test, and the toe gripping strength were significantly improved; after 12 months (85 persons), knee extension muscle strength was significantly improved although the single-leg standing, the 10m walking time period, and the toe gripping strength became approximately equal to those in the initial stage.

In a research paper authored by Shinjiro TOMITA, et al, titled "Short-term effect of locomotion training", Orthopedics & Traumatology 65; (4), pp 813-814, 2016, West-Japanese Society of Orthopedics & Traumatology, it is shown that; in the two-step test, before LT, a young-old group had 0.90, and an old-old group had 1.07 (in terms of a two-step value adjusted by body height), i.e., the stride of the young-old group is significantly greater than that of the old-old group (p value < 0.05), and, after LT, in both the two groups, the stride tended to become longer; in the stand-up test, half of each of the two groups could reduce the height of a seat from a 40 cm-height seat; and in the locomo-25 questionnaire, the score in the old-old group was changed from 45.7 to 35.3, i.e., a significant improvement in score was observed (p value < 0.02).

Thus, assuming that the locomotion training is determined as the preventive interventional action, and the amount of the locomotion training is determined as the intervention amount, it can be considered that, as the intervention amount becomes larger, or becomes closer to a proper amount, the health degree is further improved. In this case, the terminal device 100 can acquire the exercise amount of the locomotion training as the intervention amount, by communicating with an activity amount meter (which may be a wearable terminal) which has measured the exercise amount of the locomotion training, via the input-output I/F 102 in a wireless or wired manner. Alternatively, the terminal device 100 may be configured to accept an input of the exercise amount of the locomotion training from the user via the user I/F 103.

In a research paper authored by Yuichi NISHIKAWA, et al., titled "The effect of a portable electrical muscle stimulation device at home on muscle strength and activation patterns in locomotive syndrome patients: A randomized control trial", Journal of Electromyography and Kinesiology 45 (2019), pp 46-52, 2019, the International Society of Electrophysiology and Kinesiology, it is shown that an improvement in the two-step test as an LS index and an increase in thickness of muscles were observed as a result of usage of a portable electrical muscle stimulation device, called "SIXPAD". In the research paper, there are shown researches on intervention conducted by groups in Hiroshima, Nagoya and US, wherein it is shown that: the two-step test and the score of the locomo-25 questionnaire were improved by using the SIXPAD (23 minutes/time × 5 times/week × 8 weeks), but, if the SIXPAD is not subsequently used for 4 weeks, the score will go down to the initial value.

Thus, assuming that the usage of the SIXPAD is determined as the preventive interventional action, and the amount of usage of the SIXPAD is determined as the intervention amount, it can be considered that, as the intervention amount becomes larger, or becomes closer to a proper amount, the health degree is further improved. In this case, the terminal device 100 can acquire the amount of usage of the SIXPAD as the intervention amount, by preparing a controller of the SIXPAD capable of recording the usage amount, and communicating with the controller via the input-output I/F 102 in a wireless or wired manner. Alternatively, the terminal device 100 may be configured to accept an input of the amount of usage of the SIXPAD from the user via the user I/F 103.

In the article authored by Tomoyuki ARAI, et al., titled "The Relationship between Locomotive Syndrome and Higher-Level Competence in Community-Dwelling Elderly People", Physical Therapy Research 34(4), pp 417-422, 2019, the Society of Physical Therapy Science, it is shown that, as a result of dividing 338 elderly persons participating in voluntary group activity in the community into two group: a LS group; and a non-LS group, by the locomo-25 questionnaire; hearing motor function and higher-level competence from the two groups; and subjecting the hearing results to multiple logistic regression analysis, with a view to clarifying a relevance between locomotive syndrome of community-dwelling middle-aged or elderly persons, and each of higher-level competence and motor function, and it has been revealed that "social participation" is selected independently as a LS-related factor, an elderly person with LS deteriorates in terms of not only motor function but also higher-level competence, and elderly persons with little social participation among higher-level competences are determined as LS at a high rate. Further, it is reported that social participation of an elderly person is clearly relevant to his/her physical strength or motor function, mainly, of lower limb, because the presence or absence of participation in social activity is relevant to lower-limb movement ability such as the number of steps, the walking speed, standing ability, and the duration of single-leg standing, and LS defined as a state in which the locomotive function has deteriorated is relevant to social participation of an elderly person. It is also shown that the social activity means an activity to be performed by a group, a body of persons or a plurality of persons to support society or families, and examples thereof include: activity of an autonomous organization such as a neighborhood community association or a block association; activities for community renovation and regional security; volunteer and social service activities through hobbies or sports; activities for handing down traditional arts, industrial art or the like; and activities for livelihood support and parenting support. Here, the number of steps (e.g., steps/day) is considered to be approximately proportional to a movement distance (e.g., m/day).

Thus, assuming that the social activity is determined as the preventive interventional action, and the amount of participation in the social activity is determined as the intervention amount, it can be considered that, as the intervention amount becomes larger, or becomes closer to a proper amount, the health degree is further improved. In this case, a person involved in the social activity inputs the amount of participation in the social activity (e.g., times or hours/week) as the intervention amount, to the terminal device 100 via the user I/F 103, the input-output I/F 102, the wireless communication part 104, or the like. In the case of using the user I/F 103, when visiting user's home, the person involved in the social activity can input the amount of participation in the social activity to the terminal device 100, using a touch panel or the like. Alternatively, the user may input the amount of participation in the social activity to the terminal device 100, using a touch panel or the like, by himself/herself. In the case of using the input/output I/F 102, when the person involved in the social activity visits user's home with a mobile terminal to which the amount of participation in the social activity is input, the terminal device 100 can acquire the amount of participation in the social activity by communicating with the mobile terminal via the input/output I/F 102, e.g., in a wireless manner. In the case of using the wireless communication part 104, the person involved in the social activity can transmit the amount of participation in the social activity via the Internet to allow the terminal device 100 of the user to acquire the amount of participation in the social activity.

Alternatively, in this case, the number of steps or the movement distance may also be determined as the intervention amount, wherein it can be considered that, as the intervention amount becomes larger, or becomes closer to a proper amount, the health degree is further improved. The terminal device 100 can acquire such an intervention amount by communicating with an activity amount meter which has measured the intervention amount, via the input-output I/F 102 in a wireless or wired manner, or the terminal device 100 may be configured to accept an input of the intervention amount from the user via the user I/F 103.

In an article authored by Kazuhiro UENISHI, titled "Nutritional Aspects of Locomotive Syndrome", The Japanese Journal of Rehabilitation Medicine 2016, Vol. 53, pp 903-907, 2016, the Japanese Association of Medical Science, an explanation about LS and nutrients, particularly osteoporosis and sarcopenia, is made from a nutritional standpoint, wherein it is shown that a nutrient balance is important, and it is important to sufficiently ingest calcium, vitamin D, vitamin K, protein, etc., to prevent LS. It is also shown that vitamin D is synthesized in the skin when it is exposed to ultraviolet light. Thus, it is understood that exposure to ultraviolet light for an ultraviolet exposure duration close to a proper value is also important for preventing LS.

Thus, assuming that food intake is determined as the preventive interventional action, and the amount of intake of a specific nutrient is determined as the intervention amount, it can be considered that, as the intervention amount becomes closer to a proper amount, the health degree is further improved. In this case, the terminal device 100 can accept an input of the amount of intake of a specific nutrient (e.g., when the specific nutrient is calcium, the amount of intake of a calcium-containing food × the content rate of calcium in this food) as the intervention amount, from the user via the user I/F 103.

Alternatively, assuming that the ultraviolet exposure is determined as the preventive interventional action, and the duration of ultraviolet exposure is determined as the intervention amount, it can be considered that, as the intervention amount becomes closer to a proper amount, the health degree is further improved. In this case, the terminal device 100 can acquire the duration of ultraviolet exposure as the intervention amount, by communicating with an activity amount meter (which may be a wearable terminal) which has measured the duration of ultraviolet exposure, via the input-output I/F 102 in a wireless or wired manner.

In an article authored by Toshihiro WAKIMOTO, et al., titled "Situation of Locomotive syndrome in Japanese Young and Middle-aged Workers; Analysis including Mental Health Status", Ningen Dock International 33, pp 602-608, 2018, Japan Society of Ningen Dock, it is shown that a WHO-5 score which is an index of mental health degree can be adopted as a factor independent of the LS degree, and whether the user can have sufficient rest from sleep is relevant to the WHO-5 score. Thus, it is understood that a sleep time period is relevant to the LS degree. Here, the sleep time period may be a non-REM sleep time period, and a ratio between a REM sleep time period and the non-REM sleep time period may be used as an index representing goodness of sleep.

Thus, assuming that sleep is determined as the preventive interventional action, and the sleep time period is determined as the intervention amount, it can be considered that, as the intervention amount becomes closer to a proper amount, or becomes longer, or the health degree is further improved. In this case, the terminal device 100 can acquire the sleep time period as the intervention amount, by communicating with an activity amount meter (which may be a wearable terminal) which has measured the sleep time period, via the input-output I/F 102 in a wireless or wired manner. Alternatively, the terminal device 100 may be configured to accept an input of the sleep time period from the user via the user I/F 103.

In addition to the above, any of other types of actions known as a mean to contribute to preventing LS may be determined as the preventive interventional action, and a numerical value representing the amount thereof may be used as the intervention amount. For example, in the case where the food intake is determined as the preventive interventional action, the amount of calorie intake, an eating time period, or the adequacy degree of content of food intake may be determined as the intervention amount, wherein it can be it can be considered that, as the intervention amount becomes closer to a proper amount, the health degree is further improved. In the case where LT or normal exercise is determined as the preventive interventional action, the amount of consumed calorie, or a pulse rate may be determined as the intervention amount, wherein it can be it can be considered that, as the intervention amount becomes closer to a proper amount, the health degree is further improved. In the case where social activity is determined as the preventive interventional action, the amount of conversation may be determined as the intervention amount, wherein it can be it can be considered that, as the intervention amount becomes closer to a proper amount, or becomes larger, the health degree is further improved. The terminal device 100 can acquire such an intervention amount by communicating with a sensor device which has measured the intervention amount, via the input-output I/F 102 in a wireless or wired manner, or the terminal device 100 may be configured to accept an input of the intervention amount from the user via the user I/F 103.

Returning to the case where the health domain of concern is dementia, the rest of the related description will be continued below.

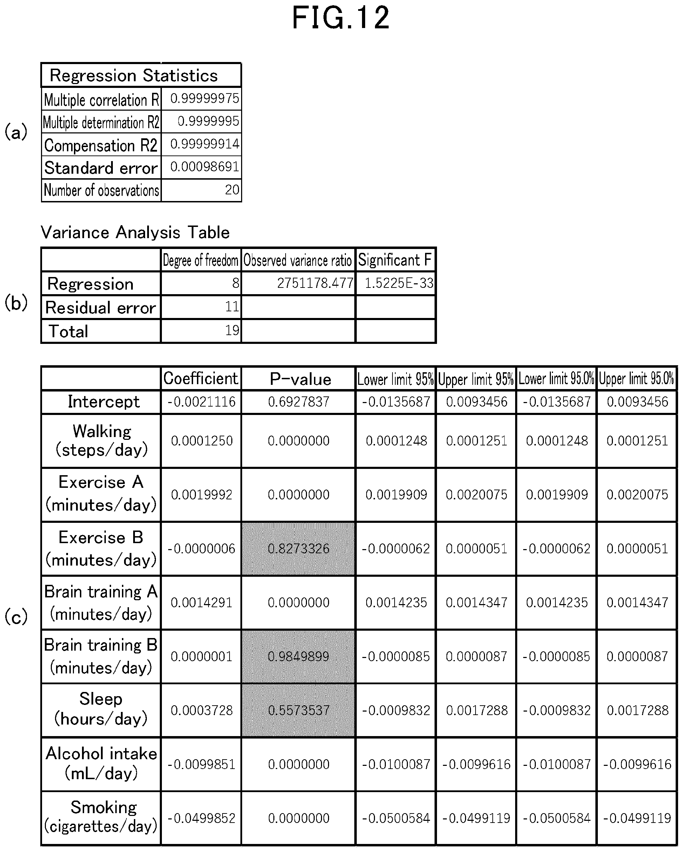

Subsequently, the control part 101 derives a correlation degree and an influence degree between the time-series intervention amount of each of the one or more preventive interventional actions and the time-series health degree of the individual (step S104). Specifically, the control part 101 derives a relevance such as the correlation degree and the influence degree, by applying a statistical method such as multivariate analysis to data about each preventive interventional action and the intervention amount thereof acquired in a time-series manner. The correlation degree is an index representing how a certain preventive interventional action is correlated with the health degree in the health domain of concern, and the influence degree is an index representing how much influence a certain preventive interventional action determined to be correlated with the health degree in the health domain of concern exerts on the health degree. For example, multivariate analysis may be used as the statistical method. Here, multiple-regression analysis is used as the multivariate analysis. FIG. 12 illustrates a table showing an example of a result of the multi-regression analysis. In the multi-regression analysis, a linear function is used as an approximation formula, and the correlation is approximated by a straight line. FIG. 12 shows a result obtained by subjecting the intervention amount of each preventive interventional action acquired in a time-series manner in the example shown in FIG. 11 to the multi-regression analysis. In FIG. 12, as a parameter representing the intervention amount of each preventive interventional action and the health degree, the amount of change (difference from the previous month) in the α2 wave is acquired monthly in a time-series manner. FIG. 12(a) shows regression statistics. In FIG. 12(a), values of "multiple correlation R" (multiple correlation coefficient), "multiple determination R2" (determination coefficient), "compensation R2", "standard error" and "the number of observations" are listed. Here, the "determination coefficient" represents the rate of a part of total variation explainable by the regression formula. Considering that the value thereof is 0.9999995, it is understood that almost all examples can be explained by the regression formula. FIG. 12(b) shows a variance analysis table presenting a test of null hypothesis. In FIG. 12(b), with regard to "regression", "residual error" and "total", values of "degree of freedom", "observed variance ratio" and "significant F" are listed. Here, the "significant F" means an upper probability of the "observed variance ratio" (F value) in an F distribution based on a two "degree of freedom": "regression" and "residual error", and is intended to test the null hypothesis that "a population correlation coefficient is zero". The value of the "significant F" in FIG. 12(b) is 1.5225E - 33 which is approximately zero, so that even when a significance level as a criterion for rejecting the null hypothesis is, e.g., 1%, the null hypothesis is rejected, and the alternative hypothesis that "the population correlation coefficient is not zero" is supported, i.e., it can be said that there is a correlation between an actually measured value and a predicted value. FIG. 12(c) shows a test result. In FIG. 12(c), with regard to respective preventive interventional actions: "walking", "exercise A", "exercise B", "brain training A", "brain training B", "sleep", "alcohol intake" and "smoking", values of "coefficient", "P-value", "lower limit 95%", "upper limit 95%", "lower limit 95.0%" and "upper limit 95.0%" are listed. In FIG. 12(c), the value of "intercept" is also listed.

In the multi-regression analysis, the P-value is relevant to the correlation degree. Specifically, the P-value represents a probability that, when the null hypothesis (i.e., a hypothesis that a certain preventive interventional action is correlated with the health degree in the health domain of concern) is correct, an extreme amount of statistics (non-correlated value) is observed. As the P-value becomes larger, the extreme amount of statistics becomes more likely to appear, i.e., the correlation degree becomes smaller. In the result in FIG. 12, the P-values of the exercise B, the brain training B and the sleep time period are in the range of 0.55 to 0.98 (see the shaded regions in FIG. 12), which shows that the correlation degree is extremely small. On the other hand, the P-values of the remaining preventive interventional actions are zero. Thus, it is understood that the correlation degree is high. The coefficient in the multi-regression analysis is a proportional constant when the correlation is approximated by a straight line, and is indicative of the magnitude of the influence. In the example in FIG. 12(c), the coefficients of the walking, the exercise A, the brain training A, the alcohol intake and the smoking determined as the preventive interventional actions are, respectively, 0.0001250, 0.0019992, 0.0014291, - 0.0099851 and -0.0499852. The value of each of the coefficients is relevant to the influence degree, so that the health degree tends to increase by the coefficient, every time the intervention amount of a corresponding one of the preventive interventional actions increases by one unit amount. With regard to the alcohol intake and the smoking, the sign of each coefficient is minus, and thus the influence degree is also minus. Thus, as the intervention amount of each of the alcohol intake and the smoking becomes smaller, the health degree becomes higher. By taking the reciprocal of each of these coefficients, it is possible to find how much the intervention amount of a corresponding one of the preventive interventional actions needs to be increased so as to increase the amount of change in the α2 wave relevant to the health degree by 1. The reciprocals of the coefficients of the walking, the exercise A, the brain training A, the alcohol intake and the smoking are, respectively, about 8000, 500, 700, - 100, and - 20. Specifically, with regard to the walking, when the number of steps is increased by about 8000 steps/day, it is expected that the amount of change in the α2 wave is increased by 1 after one month. With regard to the exercise A, when the exercise time period is increased by about 500 minutes/day, it is expected that the amount of change in the α2 wave is increased by 1 after one month. With regard to the brain training A, when the training time period is increased by about 700 minutes/day, it is expected that the amount of change in the α2 wave is increased by 1 after one month. With regard to the alcohol intake, when the amount of alcohol intake is reduced by about 100 mL/day, it is expected that the amount of change in the α2 wave is increased by 1 after one month. With regard to the smoking, when the number of cigarettes smoked is reduced by about 20 cigarettes/day, it is expected that the amount of change in the α2 wave is increased by 1 after one month. When the amount of change in the α2 wave increases, the MMSE score increases according to the relational formula: y = 1.225x - 15.95, where x denotes the MMSE score and y denotes the amount of change in the α2 wave. Therefore, in order to increase the MMSE score by 1 point, the amount of change in the α2 wave may be increased 1.225 times. Thus, with regard to the walking, when the number of steps is increased by about 9800 steps/day, it is expected that the MMSE score is increased by 1 after one month. With regard to the exercise A, when the exercise time period is increased by about 612.5 minutes/day, it is expected that the MMSE score is increased by 1 after one month. With regard to the brain training A, when the training time period is increased by about 857.5 minutes/day, it is expected that the MMSE score is increased by 1 after one month. With regard to the alcohol intake, when the amount of alcohol intake is reduced by about 122.5 mL/day, it is expected that the MMSE score is increased by 1 after one month. With regard to the smoking, when the number of cigarettes smoked is reduced by about 24.5 cigarettes/day, it is expected that the MMSE score is increased by 1 after one month.

Subsequently, the control part 101 determines, as a relevant preventive interventional action, a preventive interventional action whose correlation degree is a given value or more, among the one or more preventive interventional actions (step S105). The relevant preventive interventional action means a preventive interventional action which is deemed to be relevant to the health degree in the health domain of concern. Using the P-value which is a value relevant to the correlation degree, a preventive interventional action whose correlation degree is a given value or more can be determined as the relevant preventive interventional action by testing each preventive interventional action based on a significance level as a criterion for rejecting the null hypothesis, to reject any preventive interventional action having a low correlation degree. Specifically, the control part 101 stores therein a preliminarily-set significance level, and determines, as the relevant preventive interventional action, only a preventive interventional action whose P-value is less than the significance level. As the significance level becomes smaller, the correlation degree becomes higher, so that a state in which the correlation degree is a given value or more corresponds to a state in which the significance level is a certain value or less. The significance level is typically set to 5% or 1%. In the case where the significance level is set to 5%, the control part 101 determines a preventive interventional action whose P-value is less than 0.05, as the relevant preventive interventional action, and, on the other hand, determines and rejects a preventive interventional action whose P-value is 0.05 or more, as a non-relevant preventive interventional action. A similar operation is applied to the case where the significance level is set to 1%.

In the example in FIG. 12(c), since each of the P-values of the preventive interventional actions: the walking, the exercise A, the brain training A, the alcohol intake and the smoking, is zero, the correlation degree is higher than a value corresponding to the significance level even when the significance level is 1% or 5%. Thus, the control part 101 determines each of these preventive interventional actions, as the relevant preventive interventional action correlated with the health domain of concern. On the other hand, since the P-values of the remaining preventive interventional actions: the exercise B, the brain training B and the sleep time period are in the range of 0.55 to 0.98, the correlation degree is lower than a value corresponding to the significance level even when the significance level is 1% or 5%. Thus, the control part 101 determines each of these preventive interventional actions as a non-relevant preventive interventional action. Preferably, the control part 101 is configured to display the determined relevant preventive interventional action deemed to be relevant to the health degree in the health domain of concern, on a display or the like. In this case, more preferable, the control part 101 is configured to display the influence degree in combination. As the influence degree, the coefficient derived by the multi-regression analysis may be displayed. Alternatively, a value obtained by multiplying the reciprocal of the coefficient by 1.225 may be displayed. In this case, an intervention amount expected to cause the MMSE score to be increased by 1 point is displayed, so that it becomes possible to provide an index which is easy for the user to understand.

As above, the preventive interventional action deemed to be relevant to the health degree in the health domain of concern is derived based on the acquired biological information and the acquired intervention amount of each of the one or more preventive interventional actions, and it becomes possible to provide information about what kind of preventive interventional action is effective in improving the health degree in the health domain of concern, and what level of intervention amount leads to improvement in the health degree.

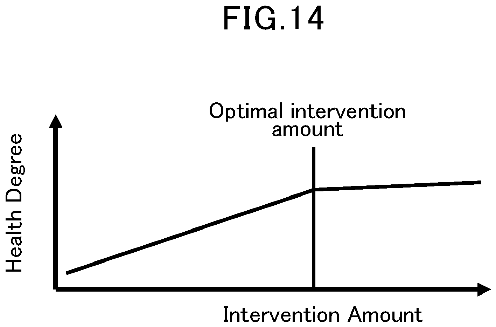

It should be noted that an optimal intervention amount may be determined to allow the preventive interventional action to more sufficiently contribute to improvement in the health degree, as follows. Specifically, based on the influence degree and the intervention amount of each of the relevant preventive interventional actions, an optimal intervention amount of each of the relevant preventive interventional actions is determined (step S106). Here, the optimal intervention amount is preferably determined as a value of the intervention amount at a time when the amount of increase in the health degree with respect to an increase in the intervention amount becomes equal to or less than a given lower limit. Specifically, with regard to the intervention value of the preventive interventional action having a plus influence degree, the health degree is improved along with an increase of this intervention value, but has an upper limit. That is, the degree of improvement in the health degree becomes gradually dull. In this situation, a sufficient improvement in the health degree cannot be expected for an increase in the intervention amount. Thus, the user can regard this intervention amount as a necessary and sufficient intervention amount, and use it as a guide for an upper limit. FIG. 14 is a graph representing an example of the relationship between the intervention amount and the health degree. FIG. 14 shows that, although the health degree increases along with an increase in the intervention amount, the degree of increase in the health degree becomes dull from a certain point. Preferably, a value of the intervention amount at a time when an increase in the health degree becomes dull despite an increase in the intervention amount, i.e., at a time when the amount of increase in the health degree with respect to an increase in the intervention amount becomes equal to or less than a given lower limit is determined as the optimal intervention amount. The point from which an increase in the health degree becomes dull can be determined by approximating the relation of the health degree to the intervention amount of only a preventive interventional action subjected to the multi-regression analysis, by an approximation formula of a curve function such as a quadratic function, a cubic function, an exponential function or a logarithmic function, and detecting it as a point at which the slope becomes a certain level or less. In a case where such a relation is approximated by an approximation formula of a linear function, the point from which an increase in the health degree becomes dull may be determined by detecting an area where the health degree tends to mostly appear on a lower side of a straight line of the approximation formula when the intervention amount is increased.

Further, a given threshold above or below which a risk of development of a given disease increases may be set in the health degree, and a time period before the health degree reaches the threshold may be predicted, based on a time-series change in the health degree (step S 107). Specifically, for example, assuming that a score of 23 in the MMSE score is set in the control part 101 as a threshold for suspecting dementia, when the health degree (MMSE score) deteriorates over time, the control part 101 derives a time period before the MMSE score reaches 23 (or a timing when the MMSE score reaches 23) from a decrease rate thereof. In this case, the user can know a timing when a risk of development of a given disease increases, and a preparation period for coping with the risk can be given to the user. Further, in order to increase the health degree (MMSE score) by a unit amount such as 1, the control part 101 may be configured to calculate how much the intervention amount of each of the preventive interventional actions should be increased, and display a result of the calculation to the user. Thus, the user can check how much the intervention amount of each of the preventive interventional actions should be increased so as to increase the health degree (MMSE score) by a given amount. This makes it easy to make a plan of implementation of each of the preventive interventional actions. Alternatively, the control part 101 may be configured to, on the assumption that the intervention amount of each of the preventive interventional actions is increased by a given value, predict a time period before the health degree reaches the threshold (or a timing when the health degree reaches the threshold) when the intervention amount is increased by the given value, and display a result of the prediction to the user. Thus, the user can check how far the timing when the risk of development of a given disease increases is shifted when the intervention amount is increased by a given amount. This makes it possible to motivate the user to increase the intervention amount of each of the preventive interventional actions. As the health degree, the MMSE score is typically used as mentioned above. Alternatively, original data (the amount of change in the α2 wave) may be directly used. Further, the original data may be converted to an index which is easy for the user to understand, such as brain age, and the index may be used.

The above embodiment has been described based on an example in which the health domain of concern is cognitive function, and the biological information is a grain wave. However, the present invention is applicable to any of various health domains of concern other than cognitive function, and the biological information may be a wide variety of information as long as they are relevant to the health degree in the health domain of concern. Therefore, the present invention may be extensively applied by using biological information relevant to each of such various health domains of concern.

### (Big Data)

In the above embodiment, an individual uses the terminal device 100 by himself/herself to acquire the biological information to access the health degree, and acquire the intervention amount of each of one or more preventive interventional actions, whereby a preventive interventional action whose correlation degree is a given value or more for the individual is determined, and the influence degree is derived. Differently, by applying a similar method to the biological information and the intervention amount of each of one or more preventive interventional actions of each of a large number of individuals, it becomes possible to determine a preventive interventional action generally correlated with the health degree of a certain health domain of concern for many persons, and the influence degree thereof. Such a method for assessing, based on one or more pieces of biological information each deemed to be relevant to a health degree in a health domain of concern of each of a plurality of individuals, a relevance between the health degree and each of one or more preventive interventional actions which are likely to contribute to maintaining or improving the health degree of each of the plurality of individuals can be implemented, e.g., by allowing a relevance server (not illustrated) connected to the terminal device 100 of each of the plurality of individuals via a network or the like to execute the following steps. The following description will be made on the assumption that the health domain of concern is cognitive function, and the biological information is a brain wave.

First of all, the biological information of each of the plurality of individuals is acquired in a time-series manner. The relevance server receives and acquires the biological data from the plurality of individual via the network in a time-series manner, wherein the biological data is acquired by the terminal device 100 owned by each of the plurality of individuals, in the same manner as that in the step S101 in the aforementioned embodiment. In this manner, the relevance server acquires so-called big data about the biological information.

Subsequently, the relevance server performs, based on the acquired biological information, an assessment of the health degree of each of the plurality of individuals in a time-series manner. Specifically, the health degree of each of the plurality of individuals corresponding to the biological data acquired in a time-series manner is derived. As with the step S 102 in the aforementioned embodiment, the biological data is brain waves each acquired in a state in which a respective one of a music-based stimulus and a photographic image-based stimulus is applied, and the health degree is the MMSE score derived from the amount of change in the α2 wave.

Subsequently, the relevance server acquires an intervention amount of each of the one or more preventive interventional actions for each of the plurality of individuals, in a time-series manner. The relevance server receives and acquires the intervention amount of each of the one or more preventive interventional actions via the network in a time-series manner, wherein the intervention amount of each of the one or more preventive interventional actions is acquired by the terminal device 100 owned by each of the plurality of individuals, in the same manner as that in the step S103 in the aforementioned embodiment. In this manner, the relevance server acquires so-called big data about the intervention amount of each of the one or more preventive interventional actions.

Subsequently, a relevance between the intervention amount of each of the one or more preventive interventional actions and the health degree is derived by machine learning using the time-series intervention amount of each of the one or more preventive interventional actions for each of the plurality of individuals and the time-series health degree of a corresponding one of the plurality of individuals. A correlation degree and an influence degree may be derived by using a statistical method such as multivariate analysis, in the same manner as that in the step S104 in the aforementioned embodiment. However, in order to cope with a situation where it is unable to predict the relevance between the intervention amount and the health degree, it is preferable to extract a rule between the intervention amount and the health degree by using machine learning such as deep learning. In the machine learning, supervised learning in a case where, with respect to each of the one or more preventive interventional actions, the intervention amount is set as an input, and the health degree is set as an output is performed by a machine learning module in the relevance server. Thus, a model in which a value corresponding to the health degree estimated when a certain intervention amount is set as an input, with respect to each of the one or more preventive interventional actions, provides a high output, will be established for various health domains of concern. In this case, when the intervention amount of a preventive interventional action having a small degree of correlation with the health degree in a certain health domain of concern is set as an input, a value output from the above model does not get higher at a point corresponding to a specific value of the health degree but distributes widely. On the other hand, when the intervention amount of a preventive interventional action having a large degree of correlation with the health degree in a certain health domain of concern is set as an input, a value output from the above model gets higher at a point corresponding to a specific value of the health degree. Thus, when the correlation degree is low, the output cannot be narrowed down to the specific value of the health degree, whereas when the correlation degree is high, the output can be narrowed down to the specific value of the health degree. Preferably, based on a difference in distribution of the output value of the model caused by a difference in the correlation degree, a preventive interventional action with a high correlation degree, in which the distribution is narrowed down to the certain health domain of concern is determined as a relevant preventive interventional action in the health domain of concern. Further, the degree of influence of the intervention of the relevant preventive interventional action on the health degree can be determined by detecting the rate of change in an output to an input of an established model. The relevant preventive interventional action corresponding to the certain health domain and the influence degree thereof each determined in the above manner are generalized information obtained under the condition that the plurality of individuals form a population, and thus can be useful information to the public for maintaining/improving the health degree.

The generalized result obtained in the above manner may be set as the relevant preventive interventional action and initial values of the influence degree thereof, in the embodiment subject to an individual. In this case, even in a state in which a relevant preventive interventional action optimal to an individual is not sufficiently determined, e.g., in a state immediately after the start of monitoring, it is possible to present, to the user, a generalized relevant preventive interventional action, etc., expected to be accurate on some level. Then, while the user continues to monitor the biological information and the intervention amount, the relevant preventive interventional action and the influence degree thereof will be changed to those corresponding to the user.

### INDUSTRIAL APPLICABILITY

The present invention is intended to, based on biological information acquired by an individual himself/herself, derive information about which of various preventive interventional actions and how much a relevant preventive interventional action is relevant to maintaining/improving the health degree in a health domain of concern of then individual, and provide the derived information to the individual. The present invention can be utilized in various devices and methods belonging to the health maintenance-related technical field and using such information.

### LIST OF REFERENCE SIGNS

100: terminal device
101: control part
101a: processor
101b: memory
101c: assessment program
101d: to-be-provided content
102: input-output interface (I/F)
103: user interface (I/F)
104: wireless communication part
200: wearable sensor

## Claims

1. A method for assessing, based on one or more pieces of biological information of an individual each deemed to be relevant to a health degree in a health domain of concern of the individual, a relevance between the health degree and each of one or more preventive interventional actions which are likely to contribute to maintaining or improving the health degree of the individual, the method comprising:
a biological information acquisition step of acquiring the biological information of the individual in a time-series manner;
a biological information assessment step of performing, based on the acquired biological information, an assessment of the health degree of the individual in a time-series manner;
an intervention amount acquisition step of acquiring an intervention amount of each of the one or more preventive interventional actions in a time-series manner;
a relevance determination step of deriving a correlation degree and an influence degree between the time-series intervention amount of each of the one or more preventive interventional actions and the time-series health degree of the individual; and
a correlation assessment step of determining, as a relevant preventive interventional action, a preventive interventional action whose correlation degree is a given value or more, among the one or more preventive interventional actions.

2. The method according to claim 1, which further comprises an intervention amount optimization step of determining an optimal intervention amount of the relevant preventive interventional action, based on respective values of the influence degree and the intervention amount in the relevant preventive interventional action.

3. The method according to claim 2, wherein the optimal intervention amount is determined as a value of the intervention amount at a time when an amount of increase in the health degree with respect to an increase in the intervention amount becomes equal to or less than a given lower limit.

4. The method according to any one of claims 1 to 3, wherein the health degree has a given threshold above or below which a risk of development of a given disease increases, and wherein the method further comprises a disease prediction step of predicting a time period before the health degree reaches the threshold, based on a time-series change in the health degree.

5. The method according to any one of claims 1 to 4, wherein the correlation degree and the influence degree are derived by a statistical method.

6. The method according to any one of claims 1 to 5, wherein the intervention amount is an activity amount of a given activity of the individual.

7. The method according to any one of claims 1 to 6, wherein the biological information of the individual includes a brain wave, and the health degree of the individual relates to a cognition function.

8. The method according to claim 7, wherein the brain wave is acquired in a state in which a specific stimulus is applied to the individual.

9. The method according to claim 8, wherein the brain wave is composed of two types of brain waves acquired in a state in which two types of specific stimuli are applied to the individual respectively, and wherein the health degree of the individual related to the cognition function is determined based on a difference in a rate of an intensity of a specific frequency component to a total intensity between the two types of acquired brain waves.

10. The method according to claim 9, wherein the two types of stimuli are music and photographic image, and wherein the specific frequency component of the brain waves corresponds to an α2 wave.

11. The method according to claim 8 or 9, wherein the stimulus is selected from the group consisting of stimulation-free condition during wakefulness and rest, stimulation-free condition during sleep, sound, music, printed image, photographic image, video image, questionnaire, quiz, game and brain training.

12. The method according to any one of claims 1 to 11, wherein the biological information of the individual is acquired by a wearable terminal.

13. The method according to any one of claims 1 to 12, wherein the intervention amount includes at least one selected from the group consisting of:
an amount of walking;
an exercise amount of a given exercise;
an amount of implementation of a given cognitive training;
a sleep time period;
an amount of alcohol intake;
a number of cigarettes smoked;
an amount of ingested nutrients;
an amount of medication/supplement/health food intake;
an amount of auditory stimulation by a given sound or music piece;
an amount of visual stimulation by a given still or moving image; and
an amount of electrical or magnetic stimulation to the brain.

14. A device for assessing, based on one or more pieces of biological information of an individual each deemed to be relevant to a health degree in a health domain of concern of the individual, a relevance between the health degree and each of one or more preventive interventional actions which are likely to contribute to maintaining or improving the health degree of the individual, the device comprising:
a biological information acquisition part to acquire the biological information of the individual in a time-series manner;
a biological information assessment part to perform, based on the acquired biological information, an assessment of the health degree of the individual in a time-series manner;
an intervention amount acquisition part to acquire an intervention amount of each of the one or more preventive interventional actions in a time-series manner;
a relevance determination part to derive a correlation degree and an influence degree between the time-series intervention amount of each of the one or more preventive interventional actions and the time-series health degree of the individual; and
a correlation assessment part to determine, as a relevant preventive interventional action, a preventive interventional action whose correlation degree is a given value or more, among the one or more preventive interventional actions.

15. A computer program configured to, when executed by a computer, cause the computer to configure a device for assessing, based on one or more pieces of biological information of an individual each deemed to be relevant to a health degree in a health domain of concern of the individual, a relevance between the health degree and each of one or more preventive interventional actions which are likely to contribute to maintaining or improving the health degree of the individual, the device comprising:
a biological information acquisition part to acquire the biological information of the individual in a time-series manner;
a biological information assessment part to perform, based on the acquired biological information, an assessment of the health degree of the individual in a time-series manner;
an intervention amount acquisition part to acquire an intervention amount of each of the one or more preventive interventional actions in a time-series manner;
a relevance determination part to derive a correlation degree and an influence degree between the time-series intervention amount of each of the one or more preventive interventional actions and the time-series health degree of the individual; and
a correlation assessment part to determine, as a relevant preventive interventional action, a preventive interventional action whose correlation degree is a given value or more, among the one or more preventive interventional actions.

16. A method for assessing, based on one or more pieces of biological information of each of a plurality of individuals each deemed to be relevant to a health degree in a health domain of concern of each of the plurality of individuals, a relevance between the health degree and each of one or more preventive interventional actions which are likely to contribute to maintaining or improving the health degree of each of the plurality of individuals, the method comprising:
a biological information acquisition step of acquiring the biological information of each of the plurality of individuals in a time-series manner;
a biological information assessment step of performing, based on the acquired biological information, an assessment of the health degree of each of the plurality of individuals in a time-series manner;
an intervention amount acquisition step of acquiring an intervention amount of each of the one or more preventive interventional actions for each of the plurality of individuals, in a time-series manner; and
a relevance determination step of deriving a relevance between each of the one or more preventive interventional actions and the health degree, by machine learning using the time-series intervention amount of each of the one or more preventive interventional actions for each of the plurality of individuals and the time-series health degree of a corresponding one of the plurality of individuals.

17. The method according to any one of claims 1 to 6, wherein
the biological information of the individual includes one or more selected from the group consisting of walking speed, acceleration, and a pulse wave-related parameter (including pulse wave velocity), and
the health degree of the individual relates to locomotive syndrome.

18. The method according to claim 17, wherein the biological information of the individual is acquired by a wearable terminal.

19. The method according to claim 17 or 18, wherein the intervention amount includes at least one selected from the group consisting of:
a training amount of locomotion training;
an amount of exercise and an amount of usage of a heating sheet;
an adequacy degree of dietary energy intake;
a food intake diversity score;
an amount of usage of a portable electrical muscle stimulation device;
an amount of aerobic exercise;
an amount of participation in social activity;
a number of steps;
a movement distance;
a duration of ultraviolet exposure;
an amount of intake of a specific nutrient;
an eating time period;
an adequacy degree of content of food intake;
an amount of calorie intake;
an amount of conversation;
an amount of sleep;
an amount of consumed calorie; and
a pulse rate.
